(19)

Europäisches
Patentamt
European
Patent Office
Office européen
des brevets

(11) **EP 2 982 363 A1**

(12) **EUROPEAN PATENT APPLICATION**

(43) Date of publication:
**10.02.2016   Bulletin 2016/06**

(51) Int Cl.:
***A61K 8/29*** *(2006.01)*          ***A61Q 17/04*** *(2006.01)*
***A61K 8/04*** *(2006.01)*

(21) Application number: **14179746.4**

(22) Date of filing: **04.08.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Omya International AG
4665 Oftringen (CH)**

(72) Inventor: **Häusler, Beatrix
5722 Gränichen (CH)**

(74) Representative: **Glas, Holger
Maiwald Patentanwalts GmbH
Elisenhof
Elisenstrasse 3
80335 München (DE)**

(54) **Two component system for cosmetic formulations**

(57)    The present invention relates to a water based dispersion, an oil based dispersion, a base formulation consisting of the water based dispersion and the oil based dispersion, a cosmetic formulation comprising the base formulation as well as a method for preparing a cosmetic formulation having UV-A and/or UV-B protection and an use of a water based dispersion and/or an oil based dispersion or a base formulation in cosmetic formulations.

EP 2 982 363 A1

**Description**

The present invention relates to a water based dispersion, an oil based dispersion, a base formulation consisting of the water based dispersion and the oil based dispersion, a cosmetic formulation comprising the base formulation as well as a method for preparing a cosmetic formulation having UV-A and/or UV-B protection and an use of a water based dispersion and/or an oil based dispersion or a base formulation in cosmetic formulations.

It is well known that the ultraviolet portion of the sun's spectrum has a damaging effect on the human skin. In particular, the ultraviolet B (UV-B) radiation ranges from 280 to 315 nm and is considered as directly damaging the DNA and causes sunburn. In contrast thereto, the ultraviolet A (UV-A) radiation ranges from 315 to 400 nm and was considered as being less damaging to the skin than the UV-B radiation for a long time. However, it is meanwhile known that the UV-A radiation causes actually more damages to the skin than UV-B radiation. Such damage is typically generated by the formation of free radicals and other reactive species developed through phototoxic reactions in the epidermis and dermis of the skin. As a consequence, the UV-A radiation is now considered as a main factor in the development of chronic light-induced alterations such as premature ageing of the skin and thus in the development of skin cancer.

Therefore, it is becoming increasingly important to protect at least the part of the skin which is exposed to sun light against UV-B as well as UV-A radiation. In the art several attempts have been made to provide sunscreens having UV protection. For example, EP 2 266 527 A2 refers to a cosmetic composition comprising (a) at least an oil soluble UV filter containing at least an alkyl- and/or alkoxy substituted dibenzoylmethane derivative; (b) polysilicone-15; (c) at least a water-soluble UV filter comprising a derivative of benzimidazole sulfonic acid; and (d) at least a substance comprising 6,7-disubstituted 2,2-dialkyl chromane compound (II) or 6,7-disubstituted 2,2-dialkyl chromene compound (III), where the composition excludes a composition comprising e.g. glyceryl stearate, stearic acid, squalane, silicon dioxide particle, iron oxide and water. US 5,916,544 A refers to a sunscreen concentrate in the form of a stable, uniform dispersion comprising, by weight:

(a) about 30-65% of ultrafine titanium dioxide or zinc oxide, or mixtures thereof,
(b) about 30-70% of an organic sunscreen agent, and (c) about 0.25-25% of a dispersing aid which is polyvinylpyrrolidone alkylated with a $C_4$ -$C_{36}$ alpha-olefin. Suitable organic sunscreen agents include cinoxate(2-ethoxyethyl-p-methoxycinnamate); diethanolamine-p-methoxycinnamate; digalloyl trioleate ethyl 4-bis(hydroxypropyl)aminobenzoate; ethylhexyl-p-methoxy-cinnamate; 2-ethylhexyl salicylate; glyceryl aminobenzoate; homosalate(3,3,5-trimethylcyclohexyl salicylate); triethanolamine salicylate; 2-phenyl-benzimidazole-5-sulfonic acid; sulisobenzone(2-hydroxy-4-methoxybenzophenone-5-sulfonic acid); Padimate A (amyl p-dimethylaminobenzoate); Padimate O (octyl dimethyl para aminobenzoate); and menthyl anthranilate, and mixtures thereof. EP 1 068 866 A2 refers to the use of micronized organic ultraviolet (UV) filters for inhibiting tanning and giving lighter appearance to human skin and hair. It is described that compositions obtained by melting one or more organic UV filters and one or more inorganic micro-pigments such as $TiO_2$, ZnO, iron oxides or other inorganic oxides, mica, or other suitable inorganic minerals, and also Ti, alkaline earth metal or zinc salts of organic acids may be used. US 6,749,838 B1 relates to a sunscreen cosmetic obtained by means of an incorporation of a composite powder of a micro particle zinc oxide having a particle size of 0.1 $\mu$m or less which is coated with a silicic anhydride at a coating rate of 5 to 30 wt % and whose surface is treated with a silicone of 3 to 12 wt %, a dispersing treatment of said composite powder in an oil or water using a media-agitating mill or a high pressure dispersing machine and/or an incorporation of a polyoxyalkylene-modified polysiloxane. WO 02/03929 A1 refers to a sunscreen formulation comprising: (a) a dispersion comprising a sunscreen active agent; and (b) a base composition comprising (i) a rheological modifying agent, and (ii) water, wherein the sunscreen composition has a particle size of less than 1000 microns and is free of surfactants. The sunscreen active agent is selected from one or more members of the group consisting of aminobenzoic acid, cinoxate, dioxybenzone, homosalate, menthyl anthranilate, octocrylene, octisalate, oxybenzone, padimate O, phenylbenzimidazole sulfonic acid, sulisobenzone, trolamine salicylate, octylmethoxycinnamate, methoxydibenzoylmethane, ethylmethoxycinnamate and butylmethoxydibenzoylmethane. US 7,470,423 B2 relates to an aqueous dispersion containing pyrogenically prepared metal oxide particles and a phosphate and/or maleic anhydride/maleate-acrylate copolymer as dispersant, a process for preparing this dispersion and its use to prepare cosmetic formulations, in particular sunscreen formulations. WO 1998/052525 A1 relates to a sunscreen formulation including conventional UV-B sunscreening agents and zinc oxide having an average particle size in the order of 150 to 800 nm. The UV-B sunscreening agent is selected from the group consisting of octyl methoxycinnamate and methylbenzylidene camphor. EP 1 544 256 A2 refers to nanoparticle titanium dioxide particles which are silica and alumina treated in the presence of citric acid and processes for preparing them.

One problem with compositions providing protection against UV-B and UV-A radiation is that in addition to the use of inorganic UV filter also organic UV filter are implemented as sunscreen in which the organic UV filter typically provides the protection against the UV-A portion of the sunlight.

However, the use of organic UV filter such as octocrylene in order to provide protection against UV-A radiation is subject to continuously increasing concerns especially because they are suspected to be a possible cause of skin irritations and allergies in sensitive persons and their potentially harmful effect on the environment. Furthermore, there is an increasing tendency on the side of the manufacturer towards storing of pre-formulated compositions which can then be easily modified such as to prepare a corresponding end formulation if needed and which avoid the handling of pulverized inorganic nano-sized titanium dioxide-containing materials and acquisition of the corresponding requisite equipment.

Therefore, there is a continuous need in the art for adequate cosmetic formulations providing sufficient UV-B and/or UV-A protection without implementing organic UV-A filter causing human health or environmental concerns. Furthermore, there is a need in the art for pre-formulated compositions which can be easily made up to the final end product and which avoid the handling of pulverized inorganic nano-sized titanium dioxide-containing materials.

Thus, it is an objective of the present invention to provide a cosmetic formulation having sufficient UV-B and/or UV-A protection. In particular, it is an objective of the present invention to provide a cosmetic formulation such as a sunscreen product, eye makeup product, lip care product, hair care product, hair styling product, nail care product, hand care product, skin care product, foundations and the like. A further objective of the present invention is to provide a cosmetic formulation being free of organic UV filter having a potentially harmful effect on the environment or a skin irritating or allergenic potential for human beings. Another objective of the present invention is to provide a cosmetic formulation having the desired properties which can be easily made-up from pre-formulated compositions. A still further objective of the present invention is to provide pre-formulated compositions which can be stored without deteriorating the optical and/or mechanical properties of the compositions and the resulting end product. Another objective of the present invention is to provide pre-formulated compositions which avoid the handling of pulverized inorganic nano-sized titanium dioxide-containing materials at an early point in the production process.

These and other objectives of the present invention can be solved by a water based dispersion, an oil based dispersion, a base formulation, a cosmetic formulation, a method for preparing the cosmetic formulation having UV-A and/or UV-B protection and the use of a water based dispersion and/or an oil based dispersion or a base formulation in cosmetic formulations as described in the present invention and defined in the claims. Advantageous embodiments of the invention are defined in the corresponding sub-claims.

According to one aspect of the present application, a water based dispersion is provided, the water based dispersion comprising

> a) from 20.0 to 60.0 wt.-%, based on the total weight of the water based dispersion, of at least one titanium dioxide-containing material,
> b) from 0.1 to 3.0 wt.-%, based on the total weight of the water based dispersion, of at least one thickener, and
> c) from 2.0 to 11.0 wt.-%, based on the total weight of the water based dispersion, of at least two additives selected from the group comprising stabilizers, chelating agents, preserving agents and wetting agents, antioxidants, and
> d) the balance up to 100.0 wt.-%, based on the total weight of the water based dispersion, is water.

According to one embodiment the at least one titanium dioxide-containing material i) comprises titanium dioxide-containing particles in crystalline form, preferably rutile, and/or ii) has a weight median particle size $d_{50}$ value in the range from 20.0 to 900.0 nm, preferably from 20.0 to 700.0 nm and most preferably from 20.0 to 550.0 nm, and/or iii) is at least one hydrophilic titanium dioxide-containing material comprising titanium dioxide-containing particles which are at least partially covered by a hydrophilic coating, preferably the at least one hydrophilic titanium dioxide-containing material comprises the hydrophilic coating in an amount of from 1.0 to 40.0 wt.-%, based on the total dry weight of the at least one hydrophilic titanium dioxide-containing material.

According to another embodiment, the titanium dioxide-containing particles of the at least one titanium dioxide-containing material i) are composed of rutile in an amount of $\geq$ 75.0 wt.-%, based on the total dry weight of the titanium dioxide-containing particles, and anatase in an amount of $\leq$ 25.0 wt.-%, based on the total dry weight of titanium dioxide in the titanium dioxide-containing particles and/or ii) comprise titanium dioxide in an amount of $\geq$ 60.0 wt.-%, preferably of $\geq$ 65.0 wt.-%, more preferably of $\geq$ 70.0 wt.-% and most preferably of $\geq$ 75.0 wt.-%, based on the total dry weight of the titanium dioxide-containing particles.

According to yet another embodiment, the at least one hydrophilic titanium dioxide-containing material comprises titanium dioxide-containing particles which are at least partially covered by a hydrophilic coating comprising at least one compound selected from the group comprising aluminium hydroxide, alumina, silica, glycerin, silicone compounds, silane and mixtures thereof.

According to one embodiment, the water based dispersion comprises as additives i) a stabilizer such as an extract from *Larix species,* preferably in an amount from 0.1 to 1.0 wt.-%, based on the total weight of the water based dispersion, and/or ii) a chelating agent such as a polyphosphate, ethylenediamine-N,N,N',N'-tetraacetic acid (EDTA), pyridine-2,6-dicarboxylic acid (DPA), diethylenetriaminepentaacetic acid (DTPA), N,N-bis(carboxymethyl)glycine (NTA), ammonium diethyldithiophosphate (DDPA), disodium ethylenediamine-tetraacetate (Na$_2$H$_2$EDTA), calcium-disodium-ethylenediamine-tetraacetate (CaNa$_2$EDTA), citric acid and salts of citric acid, sodium gluconate and mixtures thereof, preferably in an amount from 0.1 to 1.0 wt.-%, based on the total weight of the water based dispersion, and/or iii) a preserving agent such as phenoxyethanol, ethylhexylglycerin, parabens such as methyl paraben, ethyl paraben, propyl paraben, butyl paraben and mixtures thereof, benzoic acid, sodium benzoate, sorbic acid, potassium sorbate, and mixtures thereof, preferably in an amount from 0.4 to 1.5 wt.-%, based on the total weight of the water based dispersion, and/or iv) a wetting agent such as a primary alcohol, secondary alcohol, tertiary alcohol, 1,3-diol, urea and mixtures thereof, preferably in an amount from 1.4 to 7.5 wt.-%, based on the total weight of the water based dispersion, and/or v) an antioxidant such as butylhydroxyanisol (BHA), butylhydroxytoluol (BHT), gallate, carotinoid, polyphenols such as resveratrol, flavonoid and mixtures thereof, derivatives of polyphenols, ascorbic acid and salts thereof, tocopherol and salts thereof, betacarotin, ubichinon, tocotrienol, dihydroquercetin, antioxidants of natural origin and mixtures thereof.

According to another aspect of the present application, an oil based dispersion is provided, the oil based dispersion comprising

a) from 20.0 to 60.0 wt.-%, based on the total weight of the oil based dispersion, of at least one titanium dioxide-containing material, wherein the at least one titanium dioxide-containing material differs from the at least one titanium dioxide-containing material in the water based dispersion, as defined herein,
b) from 2.0 to 10.0 wt.-%, based on the total weight of the oil based dispersion, of at least one thickener,
c) from 20.0 to 40.0 wt.-%, based on the total weight of the oil based dispersion, of at least one emollient, and
d) the balance up to 100.0 wt.-%, based on the total weight of the oil based dispersion, is at least one oil.

According to one embodiment, the at least one titanium dioxide-containing material i) comprises titanium dioxide-containing particles in crystalline form preferably rutile, and/or ii) has a weight median particle size $d_{50}$ value in the range from 20.0 to 900.0 nm, preferably from 20.0 to 500.0 nm and most preferably from 20.0 to 300.0 nm, and/or iii) is at least one hydrophobic titanium dioxide-containing material comprising titanium dioxide-containing particles which are at least partially covered by a hydrophobic coating, preferably the at least one hydrophobic titanium dioxide-containing material comprises the hydrophobic coating in an amount of from 1.0 to 40.0 wt.-%, based on the total dry weight of the at least one hydrophobic titanium dioxide-containing material.

According to another embodiment, the titanium dioxide-containing particles of the at least one titanium dioxide-containing material i) are composed of rutile in an amount of ≥ 75.0 wt.-%, based on the total dry weight of the titanium dioxide-containing particles, and anatase in an amount of ≤ 25.0 wt.-%, based on the total dry weight of titanium dioxide in the titanium dioxide-containing particles and/or ii) comprise titanium dioxide in an amount of ≥ 80.0 wt.-%, preferably of ≥ 85.0 wt.-%, more preferably of ≥ 90.0 wt.-% and most preferably of ≥ 95.0 wt.-%, based on the total dry weight of the titanium dioxide-containing particles.

According to yet another embodiment, the at least one hydrophobic titanium dioxide-containing material comprises titanium dioxide-containing particles which are at least partially covered by a hydrophobic coating comprising at least one compound selected from the group comprising trimethoxy caprylsilane, triethoxy caprylsilane, dimethicone, simethicone, methicone, cyclic methicone, branched methicone, stearic acid and mixtures thereof.

According to one embodiment, the oil based dispersion comprises i) at least one thickener selected from the group comprising silicate such as magnesium silicate, aluminium silicate, silica dimethylsilicate, hydrophobic fumed silica, polyacrylic acid, salts of polyacrylic acid, derivatives of polyacrylic acid, PEG compounds such as PEG-8 myristate, PEG-30 glyceryl cocoate, PEG-80 glyceryl cocoate, PEG-15 soyamide/IPDI copolymer, PEG-40 sorbitan peroleate, PEG-150 stearate and mixtures thereof, methyl cellulose, ethyl cellulose, propyl cellulose, carboxymethylcellulose, xanthan gum, ammonium acryloyldimethyltaurate/VP copolymer and mixtures thereof, and/or ii) at least one emollient selected from the group comprising isocetylstearoylstearate, ethylhexyl stearate, octyldodecyl stearoyl stearate, isocetyl stearate, isopropyl isostearate, isostearyl isostearate, ethylhexyl hydroxystearate, ethylhexyl palmitate, isopropyl palmitate, neopentyl glycol diheptanoate, ethylhexyl isononanoate, isononyl isononanoate, cetearyl isononanoate, cetearyl octanoate, diisopropyl adipate, dicapryl adipate, diisostearylmalate, decyl oleate, isodecyl oleate, diisopropyl myristate, isostearyl neopentanoate, octyl dodecyl neopentanoate, ethylhexyl cocoate, PEG-7 glyceryl cocoate, C12-15 alkyl benzoate, C16-17 alkyl benzoate, stearyl benzoate, isostearyl benzoate, ethylhexyl benzoate, octyldodecyl benzoate, cocoglyceride, coconut alkanes, coco-caprylate/caprate and mixtures thereof, and/or iii) at least one oil selected from the group comprising alkanecoconutester, polydimethylsiloxanes, polyalkylmethylsiloxanes, silicones, vegetable oils such as palm oil, esters of vegetable oils and mixtures thereof.

According to a further aspect of the present application, a base formulation is provided, the base formulation consisting of

i) a water based dispersion, as defined herein, in an amount from 0.0 to 100.0 wt.-%, based on the total weight of the base formulation, and/or

ii) an oil based dispersion, as defined herein, in an amount from 0.0 to 100.0 wt.-%, based on the total weight of the base formulation.

According to one embodiment, the base formulation is free of octocrylene as organic UV-A filter.

According to an even further aspect of the present application, a cosmetic formulation having UV-A and/or UV-B protection is provided. The cosmetic formulation comprising

a) from 1.0 to 80.0 wt.-%, based on the total weight of the cosmetic formulation, of a base formulation as defined herein, and

b) from 20.0 to 99.0 wt.-%, based on the total weight of the cosmetic formulation, of at least one further additive selected from the group comprising emulsifier, emollients, fragrances, colorants, skin tanning compounds, stabilizers, antioxidants, pigments, preserving agents, wetting agents, oils, water and mixtures thereof.

According to one embodiment, the cosmetic formulation is selected from a sunscreen product, eye makeup product, facial makeup product, lip care product, hair care product, hair styling product, nail care product, hand care product, skin care product and mixtures thereof.

According to a still further aspect of the present application, a method for preparing a cosmetic formulation having UV-A and/or UV-B protection is provided, the method comprises at least the steps of:

a) providing a water based dispersion, as defined herein,

b) providing an oil based dispersion, as defined herein,

c) providing at least one further additive selected from the group comprising emulsifier, emollients, fragrances, colorants, stabilizers, antioxidants, pigments, preserving agents, wetting agents, oils, water and mixtures thereof, as defined herein,

d) combining the water based dispersion provided in step a) with the oil based dispersion provided in step b) such as to form a base formulation, as defined herein, and

e) combining the base formulation obtained in step d) with the at least one further additive provided in step c) such that a cosmetic formulation is formed.

According to another aspect of the present application, an use of a water based dispersion and/or an oil based dispersion or a base formulation as defined herein in cosmetic formulations is provided. Preferably, the cosmetic formulation is a sunscreen product, eye makeup product, facial makeup product, lip care product, hair care product, hair styling product, nail care product, hand care product, skin care product and mixtures thereof.

The inventors surprisingly found out that the foregoing cosmetic formulation provides excellent protection against UV-B and/or UV-A radiation without implementing organic UV filter having a potentially harmful effect on the environment or a skin irritating or allergenic potential for human beings. In addition thereto, it was surprisingly found out that the pre-formulations, i.e. the water based dispersion and the oil based dispersion, can be easily stored without deteriorating the optical and/or mechanical properties of the resulting end product and that the pre-formulations can be easily made up to the desired cosmetic formulation, while the pre-formulated compositions avoid the handling of pulverized inorganic nano-sized titanium dioxide-containing materials at an early point of the production process.

It should be understood that for the purposes of the present invention, the following terms have the following meanings:

The term "dispersion" in the meaning of the present invention refers to a system comprising a dispersing medium or solvent and at least one inorganic particulate material and/or liquid colloidal particles such as oil droplets, wherein at least a part of the particles of the at least one inorganic particulate material and/or the liquid colloidal particles are present as insoluble solids or suspended particles in the dispersing medium or solvent.

The term "water-based" dispersion refers to a dispersion wherein the dispersing medium or solvent comprises water and preferably is water.

The term "oil-based" dispersion refers to a dispersion wherein the dispersing medium or solvent comprises oil and preferably is oil.

Where the term "comprising" or "containing" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that, e.g. an embodiment must be obtained by e.g. the sequence of steps following the term "obtained" even though such a limited understanding is always included by the terms "obtained" or "defined" as a preferred embodiment.

In the following, the details and preferred embodiments of the present inventive will be described in more detail.

Water based dispersion

According to one aspect of the instant invention, a water based dispersion comprising at least one titanium dioxide-containing material is provided.

The term "at least one" titanium dioxide-containing material in the meaning of the present invention means that the titanium dioxide-containing material comprises, preferably consists of, one or more titanium dioxide-containing material(s).

In one embodiment of the present invention, the at least one titanium dioxide-containing material in the water based dispersion comprises, preferably consists of, one titanium dioxide-containing material. Alternatively, the at least one titanium dioxide-containing material comprises, preferably consists of, two or more titanium dioxide-containing materials. For example, the at least one titanium dioxide-containing material comprises, preferably consists of, two or three titanium dioxide-containing materials.

Preferably, the at least one titanium dioxide-containing material in the water based dispersion comprises, more preferably consists of, one titanium dioxide-containing material.

The at least one titanium dioxide-containing material in the water based dispersion is preferably in the form of a particulate material, and may have a particle size distribution as conventionally employed for the material(s) involved in the type of product to be produced. However, it is preferred that the at least one titanium dioxide-containing material in the water based dispersion has a weight median particle size $d_{50}$ value in the range from 20.0 to 900.0 nm. For example, the at least one titanium dioxide-containing material has a weight median particle size $d_{50}$ value from 20.0 nm to 700.0 nm and most preferably from 20.0 nm to 550.0 nm.

Throughout the present document, the "particle size" of a particulate material is described by its distribution of particle sizes. The value $d_x$ represents the diameter relative to which x % by weight of the particles have diameters less than $d_x$. This means that the $d_{25}$ value is the particle size at which 25.0 wt.-% of all particles are smaller, and the $d_{75}$ value is the particle size at which 75.0 wt.-% of all particles are smaller. The $d_{50}$ value is thus the weight median particle size, i.e. 50.0 wt.-% of all particle grains are bigger or smaller than this particle size. For the purpose of the present invention the particle size is specified as weight median particle size $d_{50}$ unless indicated otherwise. For determining the weight median particle size $d_{50}$ value, a CPS Disc Centrifuge model DC24000 of CPS Instruments Europe can be used.

It is preferred that the at least one titanium dioxide-containing material in the water based dispersion comprises titanium dioxide-containing particles in crystalline form. A preferred crystallinity of the at least one titanium dioxide-containing particles is in the range from 10.0 to 100.0 wt.-%, preferably from 40.0 to 100.0 wt.-%, more preferably above 60.0 wt.-% and most preferably above 70.0 wt.-%, based on the total dry weight of the at least one titanium dioxide-containing particles.

Preferably, the at least one titanium dioxide-containing material in the water based dispersion comprises titanium dioxide-containing particles mainly in rutile form, i.e. small amounts may also be in a brookite and/or anatase form. For example, suitable titanium dioxide-containing particles comprise at least 10.0 wt.-% of rutile structure, preferably at least 25.0 wt.-%, more preferably at least 50.0 wt.-% and most preferably at least 75.0 wt.-%, based on the total dry weight of titanium dioxide in the titanium dioxide-containing particles.

The term at least one "dry" material refers to material particles having a total surface moisture content of less than 0.5 wt.-%, preferably less than 0.2 wt.-% and more preferably less than 0.1 wt.-%, based on the total weight of the particles.

In one embodiment, the titanium dioxide-containing particles of the at least one titanium dioxide-containing material in the water based dispersion are composed of rutile in an amount of ≥ 75.0 wt.-%, based on the total dry weight of titanium dioxide in the titanium dioxide-containing particles, and anatase in an amount of ≤ 25.0 wt.-%, based on the total dry weight of titanium dioxide in the titanium dioxide-containing particles. For example, the titanium dioxide-containing particles of the at least one titanium dioxide-containing material are composed of rutile in an amount of ≥ 90.0 wt.-%, based on the total dry weight of titanium dioxide in the titanium dioxide-containing particles, and anatase in an amount of < 10.0 wt.-%, based on the total dry weight of titanium dioxide in the titanium dioxide-containing particles. Preferably, the titanium dioxide-containing particles of the at least one titanium dioxide-containing material are essentially composed of rutile, i.e. in an amount of about 100.0 wt.-%, based on the total dry weight of titanium dioxide in the titanium dioxide-containing particles.

Titanium dioxide-containing particles are usually prepared in the chloride process, in which $TiCl_4$ is oxidized to $TiO_2$ particles, or in the sulfate process, in which sulfuric acid and ore containing titanium are dissolved, and the resulting solution goes through a series of steps to yield the at least one titanium dioxide-containing particles.

The titanium dioxide-containing particles of the at least one titanium dioxide-containing material in the water based dispersion can be essentially pure titanium dioxide or may contain other metal oxides, such as silica, alumina, zirconia and the like, preferably silica. Such other metal oxides may be incorporated into the titanium dioxide-containing particles by co-oxidizing or co-precipitating titanium compounds with other metal compounds during their preparation in the chloride or sulfate process.

If other metal oxides are incorporated into the titanium dioxide-containing particles, the other metal oxides are preferably present in an amount of < 40.0 wt.-%, more preferably in an amount of < 35.0 wt.-%, even more preferably in an amount of < 30.0 wt.-% and most preferably in an amount of < 25.0 wt.-%, based on the total dry weight of the titanium dioxide-containing particles.

It is thus preferred that the titanium dioxide-containing particles of the at least one titanium dioxide-containing material in the water based dispersion comprises titanium dioxide in an amount of ≥ 60.0 wt.-%, preferably of ≥ 65.0 wt.-%, more preferably of ≥ 70.0 wt.-% and most preferably of ≥ 75.0 wt.-%, based on the total dry weight of the titanium dioxide-containing particles.

It is appreciated that the at least one titanium dioxide-containing material in the water based dispersion comprises, preferably consists of, titanium dioxide-containing particles which comprise on at least a part of the surface of said particles a hydrophilic coating.

Thus, the at least one titanium dioxide-containing material in the water based dispersion is preferably at least one hydrophilic titanium dioxide-containing material comprising titanium dioxide-containing particles which are at least partially covered by a hydrophilic coating.

Accordingly, the term "at least one" hydrophilic titanium dioxide-containing material in the meaning of the present invention means that the hydrophilic titanium dioxide-containing material comprises, preferably consists of, one or more hydrophilic titanium dioxide-containing material(s).

In one embodiment of the present invention, the at least one hydrophilic titanium dioxide-containing material in the water based dispersion comprises, preferably consists of, one hydrophilic titanium dioxide-containing material. Alternatively, the at least one hydrophilic titanium dioxide-containing material comprises, preferably consists of, two or more hydrophilic titanium dioxide-containing materials. For example, the at least one hydrophilic titanium dioxide-containing material comprises, preferably consists of, two or three hydrophilic titanium dioxide-containing materials.

Preferably, the at least one hydrophilic titanium dioxide-containing material in the water based dispersion comprises, more preferably consists of, one hydrophilic titanium dioxide-containing material.

If the at least one hydrophilic titanium dioxide-containing material comprises, preferably consists of, two or more hydrophilic titanium dioxide-containing materials, the two or more hydrophilic titanium dioxide-containing materials may differ in the titanium dioxide-containing particles and/or the hydrophilic coating on the particles.

A "hydrophilic titanium dioxide-containing material" is obtained by surface treating the titanium dioxide-containing particles with a suitable compound such that a hydrophilic coating is formed on at least a part of the accessible surface area of the titanium dioxide-containing particles.

For the purpose of the present invention, the term "hydrophilic titanium dioxide-containing material" refers to a material that comprises from 60.0 to 99.0 wt.-% of the titanium dioxide-containing particles, based on the total dry weight of the at least one hydrophilic titanium dioxide-containing material. Thus, it is preferred that the at least one hydrophilic titanium dioxide-containing material comprises the hydrophilic coating in an amount of from 1.0 to 40.0 wt.-%, based on the total dry weight of the at least one hydrophilic titanium dioxide-containing material.

For example, the at least one hydrophilic titanium dioxide-containing material comprises the titanium dioxide-containing particles in an amount of from 70.0 to 99.0 wt.-% and the hydrophilic coating in an amount of from 1.0 to 30.0 wt.-%, based on the total dry weight of the at least one hydrophilic titanium dioxide-containing material. Preferably, the at least one hydrophilic titanium dioxide-containing material comprises the titanium dioxide-containing particles in an amount of from 80.0 to 99.0 wt.-% and the hydrophilic coating in an amount of from 1.0 to 20.0 wt.-%, based on the total dry weight of the at least one hydrophilic titanium dioxide-containing material. More preferably, the at least one hydrophilic titanium dioxide-containing material comprises the titanium dioxide-containing particles in an amount of from 88.0 to 98.0 wt.-% and the hydrophilic coating in an amount of from 2.0 to 12.0 wt.-%, based on the total dry weight of the at least one hydrophilic titanium dioxide-containing material.

In one embodiment, the at least one hydrophilic titanium dioxide-containing material comprises titanium dioxide-containing particles which are at least partially covered by a hydrophilic coating comprising at least one compound selected from the group comprising aluminium hydroxide, alumina, silica, glycerin, silicone compounds, silane and mixtures thereof. Preferably, the at least one hydrophilic titanium dioxide-containing material comprises titanium dioxide-containing particles which are at least partially covered by a hydrophilic coating comprising aluminium hydroxide or glycerin. More preferably, the at least one hydrophilic titanium dioxide-containing material comprises titanium dioxide-containing particles which are at least partially covered by a hydrophilic coating comprising glycerin.

Examples of advantageous silicone compounds and silanes are compounds as described in, for example, EP 1 544 256 A2, the disclosure which is herewith incorporated by reference.

It is one requirement of the present water based dispersion that the at least one titanium dioxide-containing material, preferably the at least one hydrophilic titanium dioxide-containing material, is present from 20.0 to 60.0 wt.-%, preferably from 30.0 to 60.0 wt.-%, based on the total weight of the water based dispersion.

It is a further requirement of the instant water based dispersion that it comprises specific additives in order to provide a pre-formulated water based dispersion that can be easily made up to a desired end formulation and which can be stored without deteriorating the optical and/or mechanical properties of the pre-formulation and the end product resulting therefrom. Further, the provision of a pre-formulated water based dispersion is time-saving and avoids the handling of pulverized inorganic nano-sized titanium dioxide-containing materials.

In particular, it is required that the instant water based dispersion comprises from 0.1 to 3.0 wt.-%, based on the total weight of the water based dispersion, of at least one thickener. For example, the instant water based dispersion comprises the at least one thickener in an amount from 0.3 to 2.0 wt.-%, based on the total weight of the water based dispersion.

It is appreciated that the at least one thickener is added to give the final cosmetic formulation an aesthetically pleasing viscosity.

The term "at least one" thickener in the meaning of the present invention means that the thickener comprises, preferably consists of, one or more thickener(s).

In one embodiment of the present invention, the at least one thickener comprises, preferably consists of, one thickener. Alternatively, the at least one thickener comprises, preferably consists of, two or more thickeners. For example, the at least one thickener comprises, preferably consists of, two or three thickeners.

Preferably, the at least one thickener comprises, more preferably consists of, one thickener.

The at least one thickener is at least one known thickener. Examples of advantageous thickener are thickener based on silicate such as magnesium silicate, aluminium silicate and mixtures thereof, hydroxyethylcellulose or polyacrylamide. Preferably, the at least one thickener is based on silicate such as magnesium silicate, aluminium silicate and mixtures thereof, more preferably mixtures of magnesium and aluminium silicate.

Additionally, it is required that the water based dispersion comprises from 2.0 to 11.0 wt.-%, based on the total weight of the water based dispersion, of at least two additives selected from the group comprising stabilizers, chelating agents, preserving agents and wetting agents, antioxidants. For example, the instant water based dispersion comprises from 5.0 to 11.0 wt.-%, based on the total weight of the water based dispersion, of at least two additives selected from the group comprising stabilizers, chelating agents, preserving agents and wetting agents, antioxidants.

The term "at least two" additives in the meaning of the present invention means that the additive comprises, preferably consists of, two or more additives.

In one embodiment, the water based dispersion comprises as additives i) a stabilizer, and/or ii) a chelating agent, and/or iii) a preserving agent, and/or iv) a wetting agent, and/or v) an antioxidant. Preferably, the water based dispersion comprises as additives i) a stabilizer, and ii) a chelating agent, and iii) a preserving agent, and iv) a wetting agent. Alternatively, the water based dispersion comprises as additives i) a stabilizer, and ii) a chelating agent, and iii) a preserving agent, and iv) a wetting agent, and v) an antioxidant.

Thus, the at least two additives are preferably four additives, more preferably a stabilizer, chelating agent, preserving agent and wetting agent. Alternatively, the at least two additives are five additives, preferably a stabilizer, chelating agent, preserving agent, antioxidant and wetting agent.

If one of the at least two additives is a stabilizer, the stabilizer is preferably an extract from *Larix species.* Examples of extracts from *Larix species* include extracts from *Larix decidua* such as *Larix decidua* Mill. var. *decidua, Larix decidua* var. *polonica, Larix occidentalis, Larix gmelinii* such as *Larix gmelinii* var. *gmelinii, Larix gmelinii var. japonica, Larix gmelinii* var. *principis-rupprechtii, Larix gmelinii* var. *olgensis, Larix graffithii* such as *Larix graffithii* var. *graffithii, Larix graffithii* var. *speciosa, Larix kaempferi, Larix potaninii* such as *Larix potaninii* var. *australis, Larix potaninii* var. *macrocarpa, Larix potaninii* var. *chinensis, Larix potaninii* var. *australia, Larix potaninii* var. *himalaica, Larix potaninii* var. *potaninii, Larix sibirica, Larix laricina, Larix lyallii, Larix x eurokurilensis, Larix x eurolepis, Larix x marschlinsii, Larix x pendula* and mixtures thereof. Preferably, the stabilizer is an extract from *Larix sibirica.*

The stabilizer, if present, is preferably present in the water based dispersion in an amount from 0.1 to 1.0 wt.-%, more preferably from 0.1 to 0.7 wt.-%, based on the total weight of the water based dispersion.

If one of the at least two additives is a chelating agent, the chelating agent may be any chelating agent known to the skilled person as being suitable in cosmetic formulations. For example, the chelating agent may be added to capture iron, magnesium, calcium ions or mixtures thereof in the water based dispersion, the base formulation of the cosmetic formulation in order to prevent coloration by such ions coming from added compounds or the equipment used for preparing them.

For example, the chelating agent is selected from the group comprising a polyphosphate, ethylenediamine-N,N,N',N'-tetraacetic acid (EDTA), pyridine-2,6-dicarboxylic acid (DPA), diethylenetriaminepentaacetic acid (DTPA), N,N-bis(carboxymethyl)glycine (NTA), ammonium diethyldithiophosphate (DDPA), disodium ethylenediamine-tetraac-etate ($Na_2H_2EDTA$), calcium-disodium-ethylenediamine-tetraacetate ($CaNa_2EDTA$), citric acid and salts of citric acid, sodium gluconate and mixtures thereof Preferably, the chelating agent is a polyphosphate.

The chelating agent, if present, is preferably present in the water based dispersion in an amount from 0.1 to 1.0 wt.-%, more preferably from 0.1 to 0.7 wt.-%, based on the total weight of the water based dispersion.

If one of the at least two additives is a preserving agent, the preserving agent may be any preserving agent known to the skilled person as being suitable in cosmetic formulations.

For example, the preserving agent is selected from the group comprising phenoxyethanol, ethylhexylglycerin, parabens such as methyl paraben, ethyl paraben, propyl paraben, butyl paraben and mixtures thereof, benzoic acid, sodium benzoate, sorbic acid, potassium sorbate and mixtures thereof. Preferably, the preserving agent is selected from phenoxyethanol, ethylhexylglycerin and mixtures thereof. More preferably, the preserving agent is a mixture of phenoxyethanol and ethylhexylglycerin. For example, said mixture comprises phenoxyethanol and ethylhexylglycerin in a weight ratio of phenoxyethanol to ethylhexylglycerin from 10:1 to 1:1, more preferably from 10:1 to 3:1 and most preferably from 10:1 to 7:1 such as of about 9:1.

The preserving agent, if present, is preferably present in the water based dispersion in an amount from 0.4 to 1.5 wt.-%, more preferably from 0.7 to 1.5 wt.-%, based on the total weight of the water based dispersion.

If one of the at least two additives is a wetting agent, the wetting agent may be any wetting agent known to the skilled person as being suitable in cosmetic formulations.

For example, the wetting agent is selected from the group comprising primary alcohols such as 1-ethanol, 1-propanol, 1-butanol, isobutanol 1- pentanol, isoamyl alcohol, 2-methyl-1butanol, 1-hexanol,1-heptanol, 1-octanol, 1-nonanol, 1-decanol, 1-undecanol, 1-dodecanol, 1-tridecanol, 1-tetradecanol, 1-pentadecanol, cetyl alcohol, 1-heptadecanol, stearyl alcohol, 1-nonadecanol and mixtures thereof, secondary alcohols such as isopropanol, 2-butanol, 2-pentanol, 2-hexanol, 2-heptanol and mixtures thereof, tertiary alcohols such as tert.-butyl alcohol, tert.-amyl alcohol, 2-methyl-2-pentanol, 2-methylhexan-2-ol, 2-methylheptan-2-ol, 3-methyl-3-pentanol, 3-methyloctan-3-ol and mixtures thereof, diols such as 1,2-diols or 1,3-diols, e.g. 1,3-propandiol, urea and mixtures thereof. More preferably, the wetting agent is a diol such as a 1,3-diol, e.g. 1,3-propandiol.

The wetting agent, if present, is preferably present in the water based dispersion in an amount from 1.4 to 7.5 wt.-%, more preferably from 3.0 to 7.0 wt.-%, based on the total weight of the water based dispersion.

If one of the at least two additives is an antioxidant, the antioxidant may be any antioxidant known to the skilled person as being suitable in cosmetic formulations.

For example, the antioxidant is selected from the group comprising butylhydroxyanisol (BHA), butylhydroxytoluol (BHT), gallate, carotinoid, polyphenols such as resveratrol, flavonoid and mixtures thereof, derivatives of polyphenols, ascorbic acid and salts thereof, tocopherol and salts thereof, betacarotin, ubichinon, tocotrienol, dihydroquercetin, antioxidants of natural origin and mixtures thereof

If the antioxidant is of natural origin, the antioxidant can be e.g. a conifer extract, pinus pinaster bark extract such as Pycnogenol® from Horphag, Switzerland, and/or emblica officinalis fruit extract such as Saberry® from Sabinsa corporation, Germany.

The antioxidant, if present, is preferably present in the water based dispersion in an amount from 0.02 to 5.0 wt.-%, more preferably from 0.03 to 4.0 wt.-%, and most preferably from 0.04 to 3.0 wt.-%, based on the total weight of the water based dispersion.

It is appreciated that the balance up to 100.0 wt.-%, based on the total weight of the water based dispersion, is water. Preferably, the water is tap water, deionized water, or a mixture thereof. More preferably, the water is deionized water.

Thus, it required that the instant water based dispersion comprises, preferably consists of,

a) from 20.0 to 60.0 wt.-%, based on the total weight of the water based dispersion, of at least one titanium dioxide-containing material,
b) from 0.1 to 3.0 wt.-%, based on the total weight of the water based dispersion, of at least one thickener, and
c) from 2.0 to 11.0 wt.-%, based on the total weight of the water based dispersion, of at least two additives selected from the group comprising stabilizers, chelating agents, preserving agents and wetting agents, antioxidants, and
d) the balance up to 100 wt.-%, based on the total weight of the water based dispersion, is water.

Preferably, the instant water based dispersion comprises, more preferably consists of,

a) from 30.0 to 60.0 wt.-%, based on the total weight of the water based dispersion, of at least one titanium dioxide-containing material,
b) from 0.3 to 2.0 wt.-%, based on the total weight of the water based dispersion, of at least one thickener, and
c) from 5.0 to 11.0 wt.-%, based on the total weight of the water based dispersion, of at least two additives selected from the group comprising stabilizers, chelating agents, preserving agents and wetting agents, antioxidants, and
d) the balance up to 100.0 wt.-%, based on the total weight of the water based dispersion, is water.

In one embodiment, the instant water based dispersion comprises, preferably consists of,

a) from 20.0 to 60.0 wt.-%, based on the total weight of the water based dispersion, of at least one titanium dioxide-containing material,
b) from 0.1 to 3.0 wt.-%, based on the total weight of the water based dispersion, of at least one thickener, and
c) from 0.1 to 1.0 wt.-%, based on the total weight of the water based dispersion, of a stabilizer,
d) from 0.1 to 1.0 wt.-%, based on the total weight of the water based dispersion, of a chelating agent,
e) from 0.4 to 1.5 wt.-%, based on the total weight of the water based dispersion, of a preserving agent,
f) from 1.4 to 7.5 wt.-%, based on the total weight of the water based dispersion, of a wetting agent,
g) the balance up to 100.0 wt.-%, based on the total weight of the water based dispersion, is water.

In one embodiment, the instant water based dispersion comprises, more preferably consists of,

a) from 30.0 to 60.0 wt.-%, based on the total weight of the water based dispersion, of at least one titanium dioxide-containing material,
b) from 0.3 to 2.0 wt.-%, based on the total weight of the water based dispersion, of at least one thickener, and
c) from 0.1 to 0.7 wt.-%, based on the total weight of the water based dispersion, of a stabilizer,
d) from 0.1 to 0.7 wt.-%, based on the total weight of the water based dispersion, of a chelating agent,
e) from 0.7 to 1.5 wt.-%, based on the total weight of the water based dispersion, of a preserving agent,
f) from 3.0 to 7.0 wt.-%, based on the total weight of the water based dispersion, of a wetting agent,
g) the balance up to 100.0 wt.-%, based on the total weight of the water based dispersion, is water.

One advantage of the instant water based dispersion is that it can be stored without deteriorating the optical and/or mechanical properties of the dispersion and the resulting end product. Furthermore, the instant water based dispersion can be easily made to a cosmetic formulation and avoids the handling of a pulverized inorganic nano-sized titanium dioxide-containing material at an early point in the production process.

Oil based dispersion

According to a further aspect of the instant invention, an oil based dispersion comprising at least one titanium dioxide-containing material is provided. It is one requirement of the instant invention that the at least one titanium dioxide-containing material present in the oil based dispersion differs from the at least one titanium dioxide-containing material in the water based dispersion.
The term "at least one" titanium dioxide-containing material in the meaning of the present invention means that the titanium dioxide-containing material comprises, preferably consists of, one or more titanium dioxide-containing material(s).
In one embodiment of the present invention, the at least one titanium dioxide-containing material in the oil based dispersion comprises, preferably consists of, one titanium dioxide-containing material. Alternatively, the at least one titanium dioxide-containing material comprises, preferably consists of, two or more titanium dioxide-containing materials. For example, the at least one titanium dioxide-containing material comprises, preferably consists of, two or three titanium dioxide-containing materials.
Preferably, the at least one titanium dioxide-containing material in the oil based dispersion comprises, more preferably consists of, one titanium dioxide-containing material.
It is one requirement of the instant invention that the at least one titanium dioxide-containing material present in the oil based dispersion differs from the at least one titanium dioxide-containing material in the water based dispersion.
The term "differ" in the meaning of the present invention refers to at least one titanium dioxide-containing material in the oil based dispersion which can be differentiated from the at least one titanium dioxide-containing material in the water based dispersion by its hydrophobicity/hydrophilicity. In other words, the at least one titanium dioxide-containing material in the water based dispersion is at least one hydrophilic titanium dioxide-containing material and the at least one titanium dioxide-containing material in the oil based dispersion is at least one hydrophobic titanium dioxide-containing material.
The at least one titanium dioxide-containing material in the oil based dispersion is preferably in the form of a particulate material, and may have a particle size distribution as conventionally employed for the material(s) involved in the type of product to be produced. However, it is preferred that the at least one titanium dioxide-containing material in the oil based dispersion has a weight median particle size $d_{50}$ value in the range from 20.0 nm to 900.0 nm. For example, the at least one titanium dioxide-containing material has a weight median particle size $d_{50}$ value from 20.0 nm to 500.0 nm and most preferably from 20.0 nm to 300.0 nm.
It is preferred that the at least one titanium dioxide-containing material in the oil based dispersion comprises titanium dioxide-containing particles in crystalline form. A preferred crystallinity of the at least one titanium dioxide-containing particles is in the range from 10.0 to 100.0 wt.-%, preferably from 40.0 to 100.0 wt.-%, more preferably above 60.0 wt.-% and most preferably above 70.0 wt.-%, based on the total dry weight of the at least one titanium dioxide-containing particles.

Preferably, the at least one titanium dioxide-containing material in the oil based dispersion comprises titanium dioxide-containing particles mainly in rutile form, i.e. small amounts may also be in a brookit and/or anatase form. For example, suitable titanium dioxide-containing particles comprise at least 10.0 wt.-% of rutile structure, preferably at least 25.0 wt.-%, more preferably at least 50.0 wt.-% and most preferably at least 75.0 wt.-%, based on the total dry weight of titanium dioxide in the titanium dioxide-containing particles.

The term at least one "dry" material refers to material particles having a total surface moisture content of less than 0.5 wt.-%, preferably less than 0.2 wt.-% and more preferably less than 0.1 wt.-%, based on the total weight of the particles.

In one embodiment, the titanium dioxide-containing particles of the at least one titanium dioxide-containing material are composed of rutile in an amount of ≥ 75.0 wt.-%, based on the total dry weight of titanium dioxide in the titanium dioxide-containing particles, and anatase in an amount of ≤ 25.0 wt.-%, based on the total dry weight of titanium dioxide in the titanium dioxide-containing particles. For example, the titanium dioxide-containing particles of the at least one titanium dioxide-containing material are composed of rutile in an amount of ≥ 90.0 wt.-%, based on the total dry weight of titanium dioxide in the titanium dioxide-containing particles, and anatase in an amount of < 10.0 wt.-%, based on the total dry weight of titanium dioxide in the titanium dioxide-containing particles. Preferably, the titanium dioxide-containing particles of the at least one titanium dioxide-containing material are essentially composed of rutile, i.e. in an amount of about 100.0 wt.-%, based on the total dry weight of titanium dioxide in the titanium dioxide-containing particles.

The titanium dioxide-containing particles of the at least one titanium dioxide-containing material in the oil based dispersion can be essentially pure titanium dioxide or may contain other metal oxides, such as silica, alumina, zirconia and the like, preferably alumina. Such other metal oxides may be incorporated into the titanium dioxide-containing particles by co-oxidizing or co-precipitating titanium compounds with other metal compounds during their preparation in the chloride or sulfate process.

If other metal oxides are incorporated into the titanium dioxide-containing particles, the other metal oxides are preferably present in an amount of < 20.0 wt.-%, more preferably in an amount of < 15.0 wt.-%, even more preferably in an amount of < 10.0 wt.-% and most preferably in an amount of < 5.0 wt.-%, based on the total dry weight of the titanium dioxide-containing particles.

It is thus preferred that the titanium dioxide-containing particles of the at least one titanium dioxide-containing material comprise titanium dioxide in an amount of ≥ 80.0 wt.-%, preferably of ≥ 85.0 wt.-%, more preferably of ≥ 90.0 wt.-% and most preferably of ≥ 95.0 wt.-%, based on the total dry weight of the titanium dioxide-containing particles.

It is appreciated that the at least one titanium dioxide-containing material in the oil based dispersion comprises, preferably consists of, titanium dioxide-containing particles which comprise on at least a part of the surface of said particles a hydrophobic coating.

Thus, the at least one titanium dioxide-containing material in the oil based dispersion is preferably at least one hydrophobic titanium dioxide-containing material comprising titanium dioxide-containing particles which are at least partially covered by a hydrophobic coating.

It is thus preferred that the water based dispersion comprises a hydrophilic titanium dioxide-containing material comprising titanium dioxide-containing particles which are at least partially covered by a hydrophilic coating and the oil based dispersion comprises a hydrophobic titanium dioxide-containing material comprising titanium dioxide-containing particles which are at least partially covered by a hydrophobic coating.

Accordingly, the term "at least one" hydrophobic titanium dioxide-containing material in the meaning of the present invention means that the hydrophobic titanium dioxide-containing material comprises, preferably consists of, one or more hydrophobic titanium dioxide-containing material(s).

In one embodiment of the present invention, the at least one hydrophobic titanium dioxide-containing material in the oil based dispersion comprises, preferably consists of, one hydrophobic titanium dioxide-containing material. Alternatively, the at least one hydrophobic titanium dioxide-containing material comprises, preferably consists of, two or more hydrophobic titanium dioxide-containing materials. For example, the at least one hydrophobic titanium dioxide-containing material comprises, preferably consists of, two or three hydrophobic titanium dioxide-containing materials.

Preferably, the at least one hydrophobic titanium dioxide-containing material in the oil based dispersion comprises, more preferably consists of, one hydrophobic titanium dioxide-containing material.

If the at least one hydrophobic titanium dioxide-containing material comprises, preferably consists of, two or more hydrophobic titanium dioxide-containing materials, the two or more hydrophobic titanium dioxide-containing materials may differ in the titanium dioxide-containing particles and/or the hydrophobic coating on the particles.

A "hydrophobic titanium dioxide-containing material" is obtained by surface treating the titanium dioxide-containing particles with a suitable compound such that a hydrophobic coating is formed on at least a part of the accessible surface area of the titanium dioxide-containing particles.

For the purpose of the present invention, the term "hydrophobic titanium dioxide-containing material" refers to a material that comprises from 60.0 to 99.0 wt.-% of the titanium dioxide-containing particles, based on the total dry weight of the at least one hydrophilic titanium dioxide-containing material. Thus, it is preferred that the at least one hydrophobic titanium dioxide-containing material comprises the hydrophobic coating in an amount of from 1.0 to 40.0 wt.-%, based on the total dry weight of the at least one hydrophobic titanium dioxide-containing material.

For example, the at least one hydrophobic titanium dioxide-containing material comprises the titanium dioxide-containing particles in an amount of from 70.0 to 99.0 wt.-% and the hydrophobic coating in an amount of from 1.0 to 30.0 wt.-%, based on the total dry weight of the at least one hydrophobic titanium dioxide-containing material. Preferably, the at least one hydrophobic titanium dioxide-containing material comprises the titanium dioxide-containing particles in an amount of from 80.0 to 99.0 wt.-% and the hydrophobic coating in an amount of from 1.0 to 20.0 wt.-%, based on the total dry weight of the at least one hydrophobic titanium dioxide-containing material. More preferably, the at least one hydrophobic titanium dioxide-containing material comprises the titanium dioxide-containing particles in an amount of from 90.0 to 99.0 wt.-% and the hydrophobic coating in an amount of from 1.0 to 10.0 wt.-%, based on the total dry weight of the at least one hydrophobic titanium dioxide-containing material.

In one embodiment, the at least one hydrophobic titanium dioxide-containing material comprises titanium dioxide-containing particles which are at least partially covered by a hydrophobic coating comprising at least one compound selected from the group comprising trimethoxy caprylsilane, triethoxy caprylsilane, dimethicone, simethicone, methicone, cyclic methicone, branched methicone, stearic acid and mixtures thereof. Preferably, the at least one hydrophobic titanium dioxide-containing material comprises titanium dioxide-containing particles which are at least partially covered by a hydrophobic coating comprising triethoxy caprylsilane or stearic acid. More preferably, the at least one hydrophobic titanium dioxide-containing material comprises titanium dioxide-containing particles which are at least partially covered by a hydrophobic coating comprising triethoxy caprylsilane.

It is one requirement of the present oil based dispersion that the at least one titanium dioxide-containing material, preferably the at least one hydrophobic titanium dioxide-containing material, is present from 20.0 to 60.0 wt.-%, preferably from 30.0 to 60.0 wt.-%, based on the total weight of the oil based dispersion.

It is a further requirement of the instant oil based dispersion that it comprises specific additives in order to provide a pre-formulated oil based dispersion that can be easily made up to a desired end formulation and which can be stored without deteriorating the optical and/or mechanical properties of the pre-formulation end the end product resulting therefrom. Further, the provision of a pre-formulated oil based dispersion is time-saving and avoids the handling of pulverized inorganic nano-sized titanium dioxide-containing materials.

In particular, it is required that the instant oil based dispersion comprises from 2.0 to 10.0 wt.-%, based on the total weight of the oil based dispersion, of at least one thickener. For example, the instant oil based dispersion comprises the at least one thickener in an amount from 3.0 to 8.0 wt.-%, based on the total weight of the oil based dispersion.

It is appreciated that the at least one thickener is added to give the final cosmetic formulation an organoleptic pleasing viscosity.

The term "at least one" thickener in the meaning of the present invention means that the thickener in the oil based dispersion comprises, preferably consists of, one or more thickener(s).

In one embodiment of the present invention, the at least one thickener in the oil based dispersion comprises, preferably consists of, one thickener. Alternatively, the at least one thickener comprises, preferably consists of, two or more thickeners. For example, the at least one thickener comprises, preferably consists of, two or three thickeners.

Preferably, the at least one thickener in the oil based dispersion comprises, more preferably consists of, one thickener.

The at least one thickener in the oil based dispersion is at least one known thickener. Examples of advantageous thickener are selected from the group comprising silicate such as magnesium silicate, aluminium silicate, silica dimethylsilicate, hydrophobic fumed silica, polyacrylic acid, salts of polyacrylic acid, derivatives of polyacrylic acid, PEG compounds such as PEG-8 myristate, PEG-30 glyceryl cocoate, PEG-80 glyceryl cocoate, PEG-15 soyamide/IPDI copolymer, PEG-40 sorbitan peroleate, PEG-150 stearate and mixtures thereof, methyl cellulose, ethyl cellulose, propyl cellulose, carboxymethylcellulose, xanthan gum, ammonium acryloyldimethyltaurate/VP copolymer and mixtures thereof Preferably, the at least one thickener in the oil based dispersion is silica dimethylsilicate.

Additionally, it is required that the oil based dispersion comprises from 20.0 to 40.0 wt.-%, based on the total weight of the oil based dispersion, of at least one emollient. Preferably, the at least one emollient is present in the oil based dispersion in an amount from 22.0 to 36.0 wt.-%, based on the total weight of the oil based dispersion.

The term "at least one" emollient in the meaning of the present invention means that the emollient in the oil based dispersion comprises, preferably consists of, one or more emollient (s).

In one embodiment of the present invention, the at least one emollient in the oil based dispersion comprises, preferably consists of, one emollient. Alternatively, the at least one emollient comprises, preferably consists of, two or more emollients. For example, the at least one emollient comprises, preferably consists of, two or three emollients.

Preferably, the at least one emollient in the oil based dispersion comprises, more preferably consists of, two emollients.

The at least one emollient may be any emollient known to the skilled person as being suitable in cosmetic formulations. For example, the at least one emollient is selected from the group comprising isocetylstearoylstearate, ethylhexyl stearate, octyldodecyl stearoyl stearate, isocetyl stearate, isopropyl isostearate, isostearyl isostearate, ethylhexyl hydroxystearate, ethylhexyl palmitate, isopropyl palmitate, neopentyl glycol diheptanoate, ethylhexyl isononanoate, isononyl isononanoate, cetearyl isononanoate, cetearyl octanoate, diisopropyl adipate, dicapryl adipate, diisostearylmalate, decyl oleate, isodecyl oleate, diisopropyl myristate, isostearyl neopentanoate, octyl dodecyl neopentanoate, ethylhexyl cocoate, PEG-7 glyceril cocoate, C12-15 alkyl benzoate, C16-17 alkyl benzoate, stearyl benzoate, isostearyl benzoate, ethylhexyl benzoate, octyldodecyl benzoate, cocoglyceride, coconut alkanes, coco-caprylate/caprate and mixtures thereof. Preferably, the at least one emollient is selected from isocetylstearoylstearate, C12-15 alkyl benzoate and mixtures thereof.

It is appreciated that C12-15 alkyl benzoate is advantageously used, alone or in combination with further emollients, as C12-15 alkyl benzoate acts as SPF booster in combination with the UV-A and/or UV-B filter present in the final end product. Thus, the oil based dispersion preferably comprises C12-15 alkyl benzoate or mixtures of C12-15 alkyl benzoate with further emollients as the at least one emollient. For example, the at least one emollient is a mixture of isocetylstearoylstearate and C12-15 alkyl benzoate. For example, said mixture comprises isocetylstearoylstearate and C12-15 alkyl benzoate in a weight ratio of isocetylstearoylstearate to C12-15 alkyl benzoate from 10:1 to 1:10, more preferably from 5:1 to 1:5, even more preferably from 2:1 to 1:2 and most preferably from 1:1 to 1:2.

It is appreciated that the balance up to 100.0 wt.-%, based on the total weight of the oil based dispersion, is at least one oil.

The term "at least one" oil in the meaning of the present invention means that the oil in the oil based dispersion comprises, preferably consists of, one or more oil(s).

In one embodiment of the present invention, the at least one oil in the oil based dispersion comprises, preferably consists of, one oil. Alternatively, the at least one oil comprises, preferably consists of, two or more oils. For example, the at least one oil comprises, preferably consists of, two or three oils.

Preferably, the at least one oil in the oil based dispersion comprises, more preferably consists of, one oil.

The at least one oil may be any oil known to the skilled person as being suitable in cosmetic formulations.

For example, the at least one oil is selected from the group comprising alkanecoconutester, polydimethylsiloxanes, polyalkylmethylsiloxanes, silicones, vegetable oils such as palm oil, esters of vegetable oils and mixtures thereof. Preferably, the at least one oil is alkanecoconutester.

Thus, it required that the instant oil based dispersion comprises, preferably consists of,

a) from 20.0 to 60.0 wt.-%, based on the total weight of the oil based dispersion, of at least one titanium dioxide-containing material, wherein the at least one titanium dioxide-containing material differs from the at least one titanium dioxide-containing material in the water based dispersion,
b) from 2.0 to 10.0 wt.-%, based on the total weight of the oil based dispersion, of at least one thickener,
c) from 20.0 to 40.0 wt.-%, based on the total weight of the oil based dispersion, of at least one emollient, and
e) the balance up to 100 wt.-%, based on the total weight of the oil based dispersion, is at least one oil.

Preferably, the instant oil based dispersion comprises, more preferably consists of,

a) from 30.0 to 50.0 wt.-%, based on the total weight of the oil based dispersion, of at least one titanium dioxide-containing material, wherein the at least one titanium dioxide-containing material differs from the at least one titanium dioxide-containing material in the water based dispersion,
b) from 3.0 to 8.0 wt.-%, based on the total weight of the oil based dispersion, of at least one thickener,
c) from 22.0 to 36.0 wt.-%, based on the total weight of the oil based dispersion, of at least one emollient, and
e) the balance up to 100 wt.-%, based on the total weight of the oil based dispersion, is at least one oil.

It is appreciated that the oil based dispersion may comprise an antioxidant. The antioxidant may be any antioxidant known to the skilled person as being suitable in cosmetic formulations.

For example, the antioxidant is selected from the group comprising butylhydroxyanisol (BHA), butylhydroxytoluol (BHT), gallate, carotinoid, polyphenols such as resveratrol, flavonoid and mixtures thereof, derivatives of polyphenols, ascorbic acid and salts thereof, tocopherol and salts thereof, betacarotin, ubichinon, tocotrienol, dihydroquercetin, antioxidants of natural origin and mixtures thereof.

If the antioxidant is of natural origin, the antioxidant can be e.g. a conifer extract, pinus pinaster bark extract such as Pycnogenol® from Horphag, Switzerland, and/or emblica officinalis fruit extract such as Saberry® from Sabinsa corporation, Germany.

The antioxidant, if present, is preferably present in the oil based dispersion in an amount from 0.02 to 5.0 wt.-%, more preferably from 0.03 to 4.0 wt.-%, and most preferably from 0.04 to 3.0 wt.-%, based on the total weight of the oil based dispersion.

In one embodiment of the present invention, the oil based dispersion is stabilized by the addition of an oil/water emulsifier (o/w). For example, the oil based dispersion is stabilized by the addition of an oil/water emulsifier (o/w) having an HLB value from 8 to 15, preferably from 10 to 12.

If the oil based dispersion is stabilized with an oil/water emulsifier (o/w), the oil/water emulsifier (o/w) may be any oil/water emulsifier (o/w) known to the skilled person as being suitable in cosmetic formulations.

For example, PEG-containing emulsifiers, PEG-free emulsifiers, silicone-based emulsifiers, or emulsifiers of natural origin can be used as oil/water emulsifier (o/w).

The oil/water emulsifier (o/w), if present, is preferably present in the oil based dispersion in an amount from 1.0 to 10.0 wt.-%, more preferably from 1.0 to 8.0 wt.-%, based on the total weight of the oil based dispersion.

One advantage of the instant oil based dispersion is that it can be stored without deteriorating the optical and/or mechanical properties of the dispersion and the resulting end product. Furthermore, the instant oil based dispersion can be easily made to a cosmetic formulation and avoids the handling of a pulverized inorganic titanium dioxide-containing material at an early point in the production process.

In view of the very good results obtained with regard to the protection against UV-A and/or UV-B radiation without implementing of organic UV filter having a potentially harmful effect on the environment or a skin irritating or allergenic potential for human beings, another aspect of the present invention refers to the use of the water based dispersion and/or oil based dispersion or a base formulation in cosmetic formulations.

Preferably, the cosmetic formulation is selected from a sunscreen product, eye makeup product, facial makeup product, lip care product, hair care product, hair styling product, nail care product, hand care product, skin care product and mixtures thereof.

However, the hydrophobic titanium dioxide-containing material as defined herein may also be used in other cosmetic formulations.

Base formulation

According to another aspect of the instant invention, a base formulation is provided being prepared from the water based dispersion and/or the oil based dispersion.

With regard to the definition of the water based dispersion, the oil based dispersion and preferred embodiments thereof, reference is made to the statements provided above when discussing the technical details of the water based dispersion and the oil based dispersion of the present invention.

The base formulation thus consists of the water based dispersion in an amount from 0.0 to 100.0 wt.-%, based on the total weight of the base formulation, and/or the oil based dispersion, in an amount from 0.0 to 100.0 wt.-%, based on the total weight of the base formulation.

In one embodiment, the base formulation consists of the water based dispersion and the oil based dispersion. That is to say, the base formulation consists of the water based dispersion in an amount from 1.0 to 99.0 wt.-%, based on the total weight of the base formulation, and the oil based dispersion, in an amount from 1.0 to 99.0 wt.-%, based on the total weight of the base formulation.

Preferably, the base formulation consists of the water based dispersion in an amount from 50.0 to 99.0 wt.-%, based on the total weight of the base formulation, and the oil based dispersion, in an amount from 1.0 to 50.0 wt.-%, based on the total weight of the base formulation. More preferably, the base formulation consists of the water based dispersion in an amount from 50.0 to 90.0 wt.-%, based on the total weight of the base formulation, and the oil based dispersion, in an amount from 10.0 to 50.0 wt.-%, based on the total weight of the base formulation. Even more preferably, the base formulation consists of the water based dispersion in an amount from 50.0 to 80.0 wt.-%, based on the total weight of the base formulation, and the oil based dispersion, in an amount from 20.0 to 50.0 wt.-%, based on the total weight of the base formulation. Most preferably, the base formulation consists of the water based dispersion in an amount from 60.0 to 80.0 wt.-%, based on the total weight of the base formulation, and the oil based dispersion, in an amount from 20.0 to 40.0 wt.-%, based on the total weight of the base formulation.

In one embodiment, the base formulation is free of octocrylene as organic UV-A filter. Preferably, the base formulation is free of aminobenzoic acid, cinoxate, dioxybenzone, homosalate, menthyl anthranilate, octocrylene, octisalate, oxybenzone, padimate O, phenylbenzimidazole sulfonic acid, sulisobenzone, trolamine salicylate, octylmethoxycinnamate, methoxydibenzoylmethane, ethylmethoxycinnamate and butylmethoxydibenzoylmethane and mixtures thereof as organic UV-A filter. More preferably, the base formulation is free of organic UV-A filter.

Thus, it is appreciated that also the water based dispersion and the oil based dispersion as described above are free of octocrylene as organic UV-A filter. Preferably, the water based dispersion and the oil based dispersion as described above are free of aminobenzoic acid, cinoxate, dioxybenzone, homosalate, menthyl anthranilate, octocrylene, octisalate, oxybenzone, padimate O, phenylbenzimidazole sulfonic acid, sulisobenzone, trolamine salicylate, octylmethoxycinnamate, methoxydibenzoylmethane, ethylmethoxycinnamate and butylmethoxydibenzoylmethane and mixtures thereof as organic UV-A filter. More preferably, the water based dispersion and the oil based dispersion as described above are free of organic UV-A filter.

The base formulation may be prepared by contacting the water based dispersion with the oil based dispersion in any order in a manner known by the skilled person.

For example, the water based dispersion can be added to the oil based dispersion. Alternatively, the oil based dispersion can be added to the water based dispersion.

In one embodiment of the present invention, the base formulation is stabilized by the addition of an oil/water emulsifier (o/w). For example, the base formulation is stabilized by the addition of an oil/water emulsifier (o/w) having an HLB value from 8 to 15, preferably from 10 to 12.

If the base formulation is stabilized with an oil/water emulsifier (o/w), the oil/water emulsifier (o/w) may be any oil/water emulsifier (o/w) known to the skilled person as being suitable in cosmetic formulations.

For example, PEG-containing emulsifiers, PEG-free emulsifiers, silicone-based emulsifiers, or emulsifiers of natural origin can be used as oil/water emulsifier (o/w).

The oil/water emulsifier (o/w), if present, is preferably present in the base formulation in an amount from 1.0 to 10.0 wt.-%, more preferably from 1.0 to 8.0 wt.-%, based on the total weight of the base formulation.

It is preferred that the base formulation is prepared under conventional mixing conditions. The skilled man will adapt these mixing conditions (such as the configuration of mixing pallets and mixing speed) according to his process equipment. It is appreciated that any mixing method which would be suitable to form the base formulation, i.e. a blend of the water based dispersion and the oil based dispersion, may be used.

Cosmetic formulation

According to a further aspect of the instant invention, a cosmetic formulation having UV-A and/or UV-B protection is provided being prepared from the base formulation and at least one further additive.

With regard to the definition of the base formulation and preferred embodiments thereof, reference is made to the statements provided above when discussing the technical details of the base formulation, the water based dispersion and the oil based dispersion of the present invention.

The cosmetic formulation comprises

a) from 1.0 to 80.0 wt.-%, based on the total weight of the cosmetic formulation, of the base formulation, and
b) from 20.0 to 99.0 wt.-%, based on the total weight of the cosmetic formulation, of at least one further additive selected from the group comprising emulsifier, emollients, fragrances, colorants, skin tanning compounds, stabilizers, antioxidants, pigments, preserving agents, wetting agents, oils, water and mixtures thereof.

Preferably, the cosmetic formulation comprises

a) from 5.0 to 50.0 wt.-%, based on the total weight of the cosmetic formulation, of the base formulation, and
b) from 50.0 to 95.0 wt.-%, based on the total weight of the cosmetic formulation, of at least one further additive selected from the group comprising emulsifier, emollients, fragrances, colorants, skin tanning compounds, stabilizers, antioxidants, pigments, preserving agents, wetting agents, oils, water and mixtures thereof.

The at least one further additive selected from the group comprising emulsifier, emollients, fragrances, colorants, skin tanning compounds, stabilizers, antioxidants, pigments, preserving agents, wettings agents, oils, water and mixtures thereof may be any such additive known to the skilled person as being suitable for cosmetic formulations and which complies with cosmetic regulations. Examples of advantageous further additives are as described in, for example, Regulation EC No 1223/2009 of the European Parliament and of the Council of 30 November 2009, the disclosure which is herewith incorporated by reference.

For example, the emulsifier is preferably an ionic emulsifier, more preferably and anionic or cationic emulsifier. The emulsifier can be of natural vegetable origin e.g. polyglycerol ester or synthetic. More preferably, the emulsifier is selected from the group comprising PEG compounds, PEG-free emulsifier, silicone-based emulsifier, silicones, waxes and mixtures thereof. For example, the emulsifier is selected from the group comprising PEG compounds such as PEG-8 myristate, PEG-30 glyceryl cocoate, PEG-80 glyceryl cocoate, PEG-15 soyamide/IPDI copolymer, PEG-40 sorbitan peroleate, PEG-150 stearate and mixtures thereof, carbomer, carboxymethylcellulose, ceresin (aka mineral wax), diethanolamine (DEA), isopropyl stearate, isopropyl laurate, isopropyl palmitate, isopropyl oleate, polysorbate 20, polysorbate 60, polysorbate 80, propylene glycol, sorbitan stearate, sorbitan laurate, sorbitan palmitate, sorbitan oleate, steareth-20, triethanolamine (TEA), beeswax, candelilla wax, carnauba wax, cetearyl alcohol, cetearyl wheat bran glycosides , cetearyl wheat straw glycosides, decyl glucoside, jojoba, lecithin, vegetable glycerin, xanthan gum, coco glucoside, coconut alcohol, arachidyl alcohol, behenyl alcohol, arachidyl glucoside and mixtures thereof.

In one embodiment, the emulsifier is a mixture of coco glucoside, coconut alcohol, arachidyl alcohol, behenyl alcohol, arachidyl glucoside.

The fragrance is preferably selected from a natural and/or synthetic fragrance known as being suitable in cosmetic formulations.

The colorant is preferably selected from a natural and/or synthetic colorant, pigment or dye such as $Fe_2O_3$, ZnO, mica, bismuth oxychloride and mixtures thereof.

In one embodiment, the skin tanning compound is preferably dihydroxyacetone (DHA) and/or erythrulose. For example, the skin tanning compound is dihydroxyacetone (DHA) or erythrulose. Alternatively, the skin tanning compound is dihydroxyacetone (DHA) in combination with erythrulose.

In one embodiment, the wetting agent is preferably 1,3-propanediol.

The term "at least one" further additive in the meaning of the present invention means that the further additive comprises, preferably consists of, one or more further additive(s).

In one embodiment of the present invention, the at least one further additive comprises, preferably consists of, one further additive. Alternatively, the at least one further additive comprises, preferably consists of, two or more further additives. For example, the at least one further additive comprises, preferably consists of, two or three further additives.

Preferably, the at least one further additive comprises, more preferably consists of, two or more further additives.

If the cosmetic formulation comprises at least one further additive selected from emollients, stabilizers, preserving agents, oils, water and mixtures thereof, the at least one further additive may be the same or different to the respective additive present in the water based dispersion and/or oil based dispersion. Preferably, the at least one further additive selected from emollients, stabilizers, preserving agents, oils, water and mixtures thereof and the respective additive present in the water based dispersion and/or oil based dispersion is/are the same.

Thus, as regards the definition of the emollient, stabilizer, preserving agent, oil, water and preferred embodiments thereof, reference is made to the statements provided above when discussing the technical details of the water based dispersion and the oil based dispersion of the present invention.

For example, if the oil based dispersion comprises alkanecoconutester as the at least one oil, the oil added as the at least one further additive to prepare the cosmetic formulation is preferably the same, namely alkanecoconutester.

In one embodiment, the cosmetic formulation further comprises at least one emollient. For example, the cosmetic formulation further comprises a mixture of cocoglyceride, isononyl isononanoate, coconut alkanes and coco-caprylate/caprate as emollient.

Additionally or alternatively, the cosmetic formulation further comprises at least one thickener. For example, the cosmetic formulation further comprises ammonium acryloyldimethyltaurate/VP copolymer as thickener.

Additionally or alternatively, the cosmetic formulation further comprises at least one preserving agent. For example, the cosmetic formulation further comprises a mixture of phenoxyethanol and ethylhexylglycerin as preserving agent.

It is appreciated that the cosmetic formulation may comprise the at least one further additive and its amount in dependence of the cosmetic formulation to be prepared and/or the manufacturer's needs.

Additionally or alternatively, the cosmetic formulation may comprise the at least one titanium dioxide-containing material and its amount in dependence of the cosmetic formulation to be prepared and/or the manufacturer's needs and/or legal requirements.

For example, the cosmetic formulation may be prepared such that the total amount of the at least one titanium dioxide-containing material of the water based dispersion and the at least one titanium dioxide-containing material of the oil based dispersion is ≤ 25.0 wt.-%, based on the total weight of the cosmetic formulation. Preferably, the cosmetic formulation may be prepared such that the total amount of the at least one titanium dioxide-containing material of the water based dispersion and the at least one titanium dioxide-containing material of the oil based dispersion is from 10.0 to 25.0 wt.-%, more preferably from 10.0 to 20.0 wt.-%, based on the total weight of the cosmetic formulation.

The cosmetic formulation may be a lotion, spray, gel or other topical product. For example, the cosmetic formulation is selected from a sunscreen product, eye makeup product, facial makeup product, lip care product, hair care product, hair styling product, nail care product, hand care product, skin care product and mixtures thereof. Preferably, the cosmetic formulation is selected from a sunscreen product such as sunblock lotion or sunblock cream, suntan lotion or suntan cream, sunburn lotion or sunburn cream, sun cream or sun lotion, after sun lotion or after sun cream, sun lip balm and the like, eye makeup product such as brow liner, eye liner, eye shadow, eye mascara and the like, facial makeup product such as foundation, concealer, rouge, contour powder/creams, bronzer and the like, lip care product such as lipstick, lip balm, lip gloss, lip liner, lip plumper, lip conditioner, lip primer, lip booster and the like, hair care product such as hair serum, shampoo, dry powder shampoo, conditioner such as leave-in conditioner, hair color, hair loss products, heat protection spray and the like, hair styling product such as hair wax, hair mousse, pomade, hair gel, hair spray, styling paste, glue, hair volumizer, hair tonic and the like, nail care product or hand care product such as nail polish, lacquer, nail polish remover, nail oil and the like, skin care product such as body lotion, body cream, bronzer, hand cream, hand lotion, foot cream, face cream, face lotion, day and night creams, bb creams, cc creams, dd creams and the like, and mixtures thereof. For example, the cosmetic formulation may be a sunscreen product, eye makeup product, facial makeup product, lip care product, hair care product, hair styling product, nail care product, hand care product, skin care product which is provided with a sun protective factor such as a SPF of 10, 15, 20, 25, 30, 40, 50 or more.

Method for preparing a cosmetic formulation

According to another aspect of the instant invention, a method for preparing a cosmetic formulation having UV-A and/or UV-B protection is provided.

The method comprises at least the steps of:

a) providing a water based dispersion,
b) providing an oil based dispersion,
c) providing at least one further additive selected from the group comprising emulsifier, emollients, fragrances, colorants, stabilizers, antioxidants, pigments, preserving agents, wetting agents, oils, water and mixtures thereof,
d) combining the water based dispersion provided in step a) with the oil based dispersion provided in step b) such as to form a base formulation, and
e) combining the base formulation obtained in step d) with the at least one further additive provided in step c) such that a cosmetic formulation is formed.

With regard to the definition of the water based dispersion, the oil based dispersion, the at least one further additive, the base formulation, the cosmetic formulation and preferred embodiments thereof, reference is made to the statements provided above when discussing the technical details of the water based dispersion, the oil based dispersion, the base formulation and the cosmetic formulation of the present invention.

Preferably, the method consists of the steps:

a) providing a water based dispersion,
b) providing an oil based dispersion,
c) providing at least one further additive selected from the group comprising emulsifier, emollients, fragrances, colorants, stabilizers, antioxidants, pigments, preserving agents, wetting agents, oils, water and mixtures thereof,
d) combining the water based dispersion provided in step a) with the oil based dispersion provided in step b) such as to form a base formulation, and
e) combining the base formulation obtained in step d) with the at least one further additive provided in step c) such that a cosmetic formulation is formed.

Combining step d) may be carried out in any order in a manner known by the skilled person.

For example, combining step d) is carried out in that the water based dispersion is added to the oil based dispersion. Alternatively, combining step d) is carried out in that the oil based dispersion is added to the water based dispersion.

Preferably, combining step d) is carried out under conventional mixing conditions. The skilled man will adapt these mixing conditions (such as the configuration of mixing pallets and mixing speed) according to his process equipment. It is appreciated that any mixing method which would be suitable to form the base formulation, i.e. a blend of the water based dispersion and the oil based dispersion, may be used.

In one embodiment, combining step d) is carried out at temperatures typically used for preparing a cosmetic base formulation. Preferably, combining step d) is carried out at a temperature in the range from 30 to 60 °C, more preferably from 40 to 50 °C such as of about 45 °C.

Combining step e) may be carried out in any order in a manner known by the skilled person.

For example, combining step e) is carried out in that the base formulation is added to the at least one further additive. Alternatively, combining step e) is carried out in that the at least one further additive is added to the base formulation.

Preferably, combining step e) is carried out under conventional mixing conditions. The skilled man will adapt these mixing conditions (such as the configuration of mixing pallets and mixing speed) according to his process equipment. It is appreciated that any mixing method which would be suitable to form the cosmetic formulation, i.e. a blend of the base formulation and the at least one further additive, may be used.

In one embodiment, combining step e) is carried out at temperatures typically used for preparing a cosmetic formulation. Preferably, combining step e) is carried out at a temperature in the range from 30 to 60 °C, more preferably from 40 to 50°C such as of about 45 °C.

The following examples may additionally illustrate the invention, but are not meant to restrict the invention to the exemplified embodiments.

**EXAMPLES**

**1. Measurement processes**

The following measurement processes were used to evaluate the parameters given in the examples and claims.

**Particle size distribution (mass % particles with a diameter < *X*) and weight median diameter ($d_{50}$) of a particulate material**

Weight median grain diameter and grain diameter mass distribution of a particulate material were determined via the sedimentation process, i.e. an analysis of sedimentation behaviour in a gravitational field. The measurement was made with a CPS Disc Centrifuge model DC24000 of CPS Instruments Europe.
The method and the instrument are known to the skilled person and are commonly used to determine grain size of fillers and pigments. The measurement is carried out in an aqueous solution of 0.1 wt.-% $Na_4P_2O_7$. The samples are dispersed using a high speed stirrer and supersonics.

**Titanium dioxide content**

The titanium dioxide content is determined by x-ray fluorescence analysis.

**Sun protective factor (UVB)**

The sun protective factor was determined in accordance with ISO 24444:2010.

**UVA**

The UVA protection was determined in accordance with ISO 24443:2012.

**2. Tests**

The following water based dispersion, oil based dispersion, base formulation and cosmetic formulation were prepared:

**Water based dispersion**

A water based dispersion having a composition as described in table 1 below was prepared by mixing the components.

Table 1: Composition of the water based dispersion

| Components | Amount[#] [wt.-%] |
|---|---|
| Hydrophilic titanium dioxide* | 51.75 |
| Polyphosphate (chelating agent) | 0.5 |
| Extract from *Larix sibirica* (stabilizer) | 0.4 |
| Mg/Al silicate (thickener) | 1.2 |
| Phenoxyethanol/ethylhexylglycerin (90/10; preserving agent) | 1.0 |
| 1,3-Propanediol (wetting agent) | 5.0 |
| Water | 40.15 |

[#]: amount is based on the total amount of the water based dispersion
*: the hydrophilic titanium dioxide is a titanium dioxide being at least partially covered by a coating comprising glycerin, which is commercially available as e.g. UV Titan M040 from Sachtleben, Germany.

The water based dispersion has a thixotropic effect.

The viscosity of the water based dispersion was determined by using a Brookfield™ DV-I Prime viscometer at 25°C, and a rotation speed of 10 rpm with spindle 5. The reported viscosity values are the values detected by the instrument after 1 minute of measurement.

The viscosity of the water based dispersion immediately after production was: 3440 mPa s.

The viscosity of the water based dispersion after resting for 24hrs was: 7120 mPa s.

The hydrophilic titanium dioxide comprises titanium dioxide-containing particles comprising titanium dioxide and silica and a hydrophilic coating made from glycerin and had properties as described in table 2 below.

Table 2: Properties of the hydrophilic titanium dioxide

| | |
|---|---|
| Titanium dioxide content of the hydrophilic titanium dioxide | 70.0 - 73.0 wt.-% |
| $SiO_2$ content of the hydrophilic titanium dioxide (coating) | 17.0 wt.-% |
| Glycerin content of the hydrophilic titanium dioxide (coating) | 10.0 wt.-% |
| $d_{50}$ | 532 nm |

**Oil based dispersion**

An oil based dispersion having a composition as described in table 3 below was prepared by mixing the components.

Table 3: Composition of the oil based dispersion

| Components | Amount[#] [wt.-%] |
|---|---|
| Hydrophobic titanium dioxide* | 45.72 |
| C12-C15 alkyl benzoate (emollient) | 16.78 |
| Isocetylstearoylstearate (emollient) | 13.0 |
| Silica dimethylsilicate (thickener) | 6.5 |
| Alkanecoconutester (oil) | 18.0 |
| [#]: amount is based on the total amount of the oil based dispersion | |
| *: the hydrophobic titanium dioxide is a titanium dioxide being at least partially covered by a coating comprising triethoxy caprylsilane, which is commercially available as e.g. UV Titan M765 from Sachtleben, Germany. | |

The Brookfield viscosity of the oil based dispersion was determined at 25°C with spindle 6, at 10 rpm. Viscosity immediate after production: 48700 mPa s

Viscosity after water based dispersion rested for 24hrs: 49700 mPa s

The viscosity of the oil based dispersion was determined by using a Brookfield™ DV-I Prime viscometer at 25°C, and a rotation speed of 10 rpm with spindle 6. The reported viscosity values are the values detected by the instrument after 1 minute of measurement.

The viscosity of the oil based dispersion immediately after production was: 48700 mPa s.

The viscosity of the oil based dispersion after resting for 24hrs was: 49700 mPa s.

The hydrophobic titanium dioxide comprises titanium dioxide-containing particles comprising titanium dioxide and alumina and a hydrophobic coating made from triethoxy caprylsilane and had properties as described in table 4 below.

Table 4: Properties of the hydrophobic titanium dioxide

| | |
|---|---|
| Titanium dioxide content of the hydrophobic titanium dioxide | 87.0 - 88.0 wt.-% |
| $Al_2O_3$ content of the hydrophobic titanium dioxide | 3.0 wt.-% |
| Coating content of the hydrophobic titanium dioxide (triethoxy caprylsilane) | 9.0 wt.-% |
| $d_{50}$ | 298 nm |

**Base formulation**

A base formulation as described in table 5 below was prepared by mixing the water based dispersion and the oil based dispersion.

Table 5: Composition of the base formulation

| Components | Amount[#] [wt.-%] |
|---|---|
| Water based dispersion | 68.35 |
| Oil based dispersion | 31.65 |
| #: amount is based on the total amount of the base formulation | |

**Cosmetic formulation**

A cosmetic formulation A as described in table 6 below was prepared by mixing the water based dispersion and the oil based dispersion.

Table 6: Composition of the cosmetic formulation A

| * | Content [wt.-%][#] | Component |
|---|---|---|
| 1 | 3.00 | Coco glucoside and coconut alcohol |
| 2 | 1.00 | Arachidyl alcohol and behenyl alcohol and arachidyl glucoside |
| 3 | 5.00 | Cocoglycerides |
| 4 | 2.00 | Isononyl isononanoate |
| 5 | 3.00 | Coconut alkanes and coco-caprylate/caprate |
| 6 | 12.50 | Oil based dispersion |
| 7 | 42.65 | Water |
| 8 | 0.15 | Ammonium acryloyldimethyltaurate/VP copolymer |
| 9 | 3.00 | 1,3-propanediol |
| 10 | 0.70 | Phenoxyethanol and ethylhexylglycerin |
| 11 | 27.00 | Water based dispersion |

*: the components are available as follows: 1 is an emulsifier and is commercially available as Montanov™ S from SEPPIC, SA, France; 2 is an emulsifier and is commercially available as Montanov™ 202 from SEPPIC, SA, France; 3 is an emollient and is commercially available as Myritol 331® from BASF The Chemical Company, Germany; 4 is an emollient and is commercially available as DUB-INN from Stéarinerie Dubois, France; 5 is an emollient and is commercially available as Vegelight 1214LC from BioSynthis, France; 8 is a thickening or gelling agent and is commercially available as Aristoflex® AVC from Clariant, Switzerland; 9 is a wetting agent and is commercially available from DuPont Tate & Lyle, USA; 10 is a preserving agent and is commercially available as Euxyl® PE9010 from Schülke & Mayr, Germany; #: wt.-% is based on the total weight of the cosmetic formulation

**A) Evaluation of Sun Protection by EN ISO 24440:2010 *In Vivo* Determination of the Sun Protection Factor (SPF)**

The cosmetic formulation A was prepared and tested by the Research Institute for Reliable Results - Dermatest® GmbH, in 48008 Münster, Germany.

The testing was carried out on 10 subjects *in-vivo* according to EN ISO 24444:2010. 10 test persons were selected with respect to their skin types classified according to Fitzpatrick Scale as published in Arch Dermatol. 124; 869-871 (1988). The types selected were skin types I to III out of the types I-VI. Type I - always burns, never tans, Type II - always burns, tans minimally, Type III - burns moderately, tans uniformly.

Panel of test persons were 3 male and 7 female persons in a range of age from 24 to 53, out of which were 2 of skin type I, 6 of skin type II and 2 of skin type III.

### a) UV light source

In a Solar Light Company 601-300 Multiport Simulator the light spectrum of a Xenon arc was specifically filtered to emit in the erythemal effective range and displayed on the skin. The simulator was equipped with 6 irradiation fields for emitting simultaneously different irradiation doses.

### b) Determination of Minimal Erythemal Dose (MED)

The individual subject's MED is the shortest time of exposure that produces minimally perceptible erythema at 16 to 24 hours post irradiation. In order to determine the individual MED of the test person, a series of six test areas of one 1 cm diameter each was irradiated with 6 individual UV irradiations of different intensities 24 hours prior to the effective measurement of the SPF determination. The intensities were increased by 25% from one test area to the next. The irradiated areas were assessed 16 to 24 hours post irradiation and the MED of the unprotected skin $MED_u$ was determined. The MED serves as an indicator for the dose to be applied for the determination of the SPF. MED is defined as the irradiation energy which is needed to produce a weak but still noticeable erythema (reddened skin) with sharp edges.

### c) Main Test

The individual SPF of cosmetic formulation A and of the reference Standard P2 was determined on specific positions on the back of each test person. For this, 35 $cm^2$ areas were used for testing cosmetic formulation A and Standard P2 as prescribed by EN ISO 24444:2010. Cosmetic formulation A and Standard P2 were applied in amounts of 2 $mg/cm^2$, and irradiation with UV radiation was made 15 - 30 min after application.

The effective irradiation time was determined in relation to the MED of unprotected skin and the assumed SPF of the cosmetic formulation A. For this, $MED_u$ was multiplied with the assumed SPF of cosmetic formulation A, the results being the amount of minutes for irradiation. For formulations with an expected SPF of below 25, a geometric series of 25 % increase in radiation intensity was defined. For formulations with an expected SPF of 25 or higher, a geometric series of 12.5 % increase in radiation intensity was defined. After irradiation of the test areas the skin was protected from further exposure to UV- or sunlight. All test persons were re-examined the next day (16 to 24 hours later).

### d) Evaluation of test results

Evaluation of the test areas was performed by visual examination by a technician, 16 to 24 hours after exposure to the UV irradiation test.

$$SPF = \frac{MED_p}{MED_u}$$

$MED_p$ =Protected MED
$MED_u$ =Final unprotected MED

### e) Results

The test results for determining the SPF and $MED_p$ for the cosmetic formulation A as well as the standard P2 are outlined in table 7 below.

Table 7: Test results

| Test pers. | $MED_u$ [mJ/cm$^2$] | $MED_p$ [mJ/cm$^2$] Cosmetic formula. A | SPF Cosmetic formula. A | $MED_p$ [mJ/cm$^2$] Standard P2 | SPF Standard P2 |
|---|---|---|---|---|---|
| 1. | 25 | 654 | 26.2 | 559 | 22.4 |
| 2. | 25 | 581 | 23.2 | 447 | 17.9 |
| 3. | 33 | 864 | 26.2 | 590 | 17.9 |
| 4. | 27 | 564 | 20.9 | 386 | 14.3 |
| 5. | 25 | 581 | 23.2 | 447 | 17.9 |

(continued)

| Test pers. | MED$_u$ [mJ/cm$^2$] | MED$_p$ [mJ/cm$^2$] Cosmetic formula. A | SPF Cosmetic formula. A | MED$_p$ [mJ/cm$^2$] Standard P2 | SPF Standard P2 |
|---|---|---|---|---|---|
| 6. | 21 | 438 | 20.9 | 479 | 22.8 |
| 7. | 25 | 581 | 23.2 | 357 | 14.3 |
| 8. | 33 | 766 | 23.2 | 472 | 14.3 |
| 9. | 27 | 627 | 23.2 | 386 | 14.3 |
| 10. | 22 | 495 | 20.9 | 394 | 17.9 |

A summary of the results obtained for the cosmetic formulation A as well as the standard P2 are outlined in table 8 below.

Table 8: Summarized results

| Product | Recommended SPF | SPF category | 95% confidential interval | SPF (mean) | Standard Deviation |
|---|---|---|---|---|---|
| Cosmetic formulation A | 20 | medium protection level | 21.7-24.5 | 23.1 | 1.9 |
| Standard P2 | 15 | medium protection level | 15.1-19.7 | 17.4 | 3.2 |

It is appreciated that the SPF (mean) value is obtained by summing up the corresponding SPF values outlined in table 7 and dividing the obtained value by the number of measurements per condition, i.e. 10.

From table 8, it can be gathered that the test results for cosmetic formulation A are in line with the statistic criteria for the SPF testing method of EN ISO 24444:2010. Furthermore, the mean SPF value for Standard P2 lies within the requested and expected measuring range.

**B) In Vitro determination of UV-A protection by EN ISO 24443:2012**

The cosmetic formulation A was prepared and tested by the Research Institute for Reliable Results - Dermatest® GmbH, in 48008 Münster, Germany.

**a) Principle**

The determination of UV-A protection is based on the methodology of "Determination of sunscreen UVA photoprotection *in vitro*" according to EN ISO 24443:2012.

The determination of the UVA Protection Factor (UVAPF) provided by the test substance was determined *in vitro* in correlation to determined or claimed Sun Protection Factor. The test is based on the measurement of UV-light transmission of a thin film of material to be tested, which has been applied on a specific, surface roughened object plate (polymethylmethacrylate, Plexiglas ™, also known as PMMA-plates). Transmission was measured before and after irradiation with a specific dose of UV-light. For irradiation with a specific dose of UV-light is calculated for the substance to be tested.

The PMMA-plates were transparent for UV-light, not-fluorescent, photostable and inert against substances present in the material to be tested. The material to be tested was applied in an amount of 1.3 mg/cm$^2$ on the roughened surface of the PMMA-plate. In order to guarantee an accurate preciseness and reproducibility of the results, the surface area coated with the material to be tested should not be less than 16 cm$^2$. The testing substance was applied uniformly over the whole PMMA-plate surface and kept for 30 minutes in the dark, or acclimatisation to the environmental conditions, in order to support the formation of a standardised film of the testing substance.

The spectrophotometer used was a UV-2000S from Labsphere, Inc. in the range of 290 to 400 nm, equipped with a cooling system to cool samples to 25 °C to 35 °C. The UV-light source provided by Suntest CPS+, Atlas, USA, exhibits in the irradiation plane a spectral irradiation intensity, which corresponds as close as possible to the irradiation intensity close to the ground under the standard solar zenith angle, as defined by COLIPA (1994) or DIN 67501(1999).

The transmission of the UV-light of the sample (coated PMMA-plate) was done on different places of the coated PMMA-plate in the spectrophotometer UV-2000S. The product to be tested was applied on at least four PMMA-plates, and each PMMA-plate was measured several times at different positions in order to determine the correction factor "C", which should be in the range from 0.8 to 1.6.

Calculation of the UVA-dose "D" for the irradiation was derived from the $UVAPF_0$ value. The sample was irradiated with the whole range of the UV-light spectrum, the dose of the light, however, being dependent on the UVA-protection of the sample. The UVAPF-value of the sample is the mean of the UVAPF-values of the single tested samples.

The ratio of SPF/experimental UVAPF-value was calculated based on the assumed *in-vivo* SPF and the experimental determined *in vitro* UVAPF.

**b) Results**

The test results for determining the UVAPF for the cosmetic formulation A as well as the standard P2 are outlined in table 9 below.

Table 9: Test results

| Sample | assumed *in-vivo* SPF | applied dose | UVAPF | UVAPF/SPF | Number of plates |
|---|---|---|---|---|---|
| Cosmetic formulation A | 30 | 10.84 J/cm$^2$ | 9.05 | 0.302 | 4 |
| Standard P2 | 16 | 16.71 J/cm$^2$ | 14.61 | 0.913 | 4 |

It can be gathered from table 9 that the cosmetic formulation A has a UVA-protection factor of 30.2 % of the corresponding UVB-protection value.

**Claims**

1. Water based dispersion comprising

    a) from 20.0 to 60.0 wt.-%, based on the total weight of the water based dispersion, of at least one titanium dioxide-containing material,
    b) from 0.1 to 3.0 wt.-%, based on the total weight of the water based dispersion, of at least one thickener, and
    c) from 2.0 to 11.0 wt.-%, based on the total weight of the water based dispersion, of at least two additives selected from the group comprising stabilizers, chelating agents, preserving agents and wetting agents, antioxidants, and
    d) the balance up to 100.0 wt.-%, based on the total weight of the water based dispersion, is water.

2. Water based dispersion according to claim 1, wherein the at least one titanium dioxide-containing material

    i) comprises titanium dioxide-containing particles in crystalline form, preferably rutile, and/or
    ii) has a weight median particle size $d_{50}$ value in the range from 20.0 to 900.0 nm, preferably from 20.0 to 700.0 nm and most preferably from 20.0 to 550.0 nm, and/or
    iii) is at least one hydrophilic titanium dioxide-containing material comprising titanium dioxide-containing particles which are at least partially covered by a hydrophilic coating, preferably the at least one hydrophilic titanium dioxide-containing material comprises the hydrophilic coating in an amount of from 1.0 to 40.0 wt.-%, based on the total dry weight of the at least one hydrophilic titanium dioxide-containing material.

3. Water based dispersion according to claim 2, wherein the titanium dioxide-containing particles of the at least one titanium dioxide-containing material

    i) are composed of rutile in an amount of ≥ 75.0 wt.-%, based on the total dry weight of the titanium dioxide-containing particles, and anatase in an amount of ≤ 25.0 wt.-%, based on the total dry weight of titanium dioxide in the titanium dioxide-containing particles, and/or
    ii) comprise titanium dioxide in an amount of ≥ 60.0 wt.-%, preferably of ≥ 65.0 wt.-%, more preferably of ≥ 70.0 wt.-% and most preferably of ≥ 75.0 wt.-%, based on the total dry weight of the titanium dioxide-containing particles.

4. Water based dispersion according to claim 2 or 3, wherein the at least one hydrophilic titanium dioxide-containing material comprises titanium dioxide-containing particles which are at least partially covered by a hydrophilic coating comprising at least one compound selected from the group comprising aluminium hydroxide, alumina, silica, glycerin, silicone compounds, silane and mixtures thereof.

**5.** Water based dispersion according to any one of claims 1 to 4, wherein the water based dispersion comprises as additives

> i) a stabilizer such as an extract from *Larix species,* preferably in an amount from 0.1 to 1.0 wt.-%, based on the total weight of the water based dispersion, and/or
> ii) a chelating agent such as a polyphosphate, ethylenediamine-N,N,N',N'-tetraacetic acid (EDTA), pyridine-2,6-dicarboxylic acid (DPA), diethylenetriaminepentaacetic acid (DTPA), N,N-bis(carboxymethyl)glycine (NTA), ammonium diethyldithiophosphate (DDPA), disodium ethylenediamine-tetraacetate ($Na_2H_2EDTA$), calcium-disodium-ethylenediamine-tetraacetate ($CaNa_2EDTA$), citric acid and salts of citric acid, sodium gluconate and mixtures thereof, preferably in an amount from 0.1 to 1.0 wt.-%, based on the total weight of the water based dispersion, and/or
> iii) a preserving agent such as phenoxyethanol, ethylhexylglycerin, parabens such as methyl paraben, ethyl paraben, propyl paraben, butyl paraben and mixtures thereof, benzoic acid, sodium benzoate, sorbic acid, potassium sorbate, and mixtures thereof, preferably in an amount from 0.4 to 1.5 wt.-%, based on the total weight of the water based dispersion, and/or
> iv) a wetting agent such as a primary alcohol, secondary alcohol, tertiary alcohol, 1,3-diol, urea and mixtures thereof, preferably in an amount from 1.4 to 7.5 wt.-%, based on the total weight of the water based dispersion, and/or
> v) an antioxidant such as butylhydroxyanisol (BHA), butylhydroxytoluol (BHT), gallate, carotinoid, polyphenols such as resveratrol, flavonoid and mixtures thereof, derivatives of polyphenols, ascorbic acid and salts thereof, tocopherol and salts thereof, betacarotin, ubichinon, tocotrienol, dihydroquercetin, antioxidants of natural origin and mixtures thereof.

**6.** Oil based dispersion comprising

> a) from 20.0 to 60.0 wt.-%, based on the total weight of the oil based dispersion, of at least one titanium dioxide-containing material, wherein the at least one titanium dioxide-containing material differs from the at least one titanium dioxide-containing material in the water based dispersion as defined in any one of claims 1 to 5,
> b) from 2.0 to 10.0 wt.-%, based on the total weight of the oil based dispersion, of at least one thickener,
> c) from 20.0 to 40.0 wt.-%, based on the total weight of the oil based dispersion, of at least one emollient, and
> d) the balance up to 100.0 wt.-%, based on the total weight of the oil based dispersion, is at least one oil.

**7.** Oil based dispersion according to claim 6, wherein the at least one titanium dioxide-containing material

> i) comprises titanium dioxide-containing particles in crystalline form preferably rutile, and/or
> ii) has a weight median particle size $d_{50}$ value in the range from 20.0 to 900.0 nm, preferably from 20.0 to 500.0 nm and most preferably from 20.0 to 300.0 nm, and/or
> iii) is at least one hydrophobic titanium dioxide-containing material comprising titanium dioxide-containing particles which are at least partially covered by a hydrophobic coating, preferably the at least one hydrophobic titanium dioxide-containing material comprises the hydrophobic coating in an amount of from 1.0 to 40.0 wt.-%, based on the total dry weight of the at least one hydrophobic titanium dioxide-containing material.

**8.** Oil based dispersion according to claim 7, wherein the titanium dioxide-containing particles of the at least one titanium dioxide-containing material

> i) are composed of rutile in an amount of $\geq$ 75.0 wt.-%, based on the total dry weight of the titanium dioxide-containing particles, and anatase in an amount of $\leq$ 25.0 wt.-%, based on the total dry weight of titanium dioxide in the titanium dioxide-containing particles, and/or
> ii) comprise titanium dioxide in an amount of $\geq$ 80.0 wt.-%, preferably of $\geq$ 85.0 wt.-%, more preferably of $\geq$ 90.0 wt.-% and most preferably of $\geq$ 95.0 wt.-%, based on the total dry weight of the titanium dioxide-containing particles.

**9.** Oil based dispersion according to claim 7 or 8, wherein the at least one hydrophobic titanium dioxide-containing material comprises titanium dioxide-containing particles which are at least partially covered by a hydrophobic coating comprising at least one compound selected from the group comprising trimethoxy caprylsilane, triethoxy caprylsilane, dimethicone, simethicone, methicone, cyclic methicone, branched methicone, stearic acid and mixtures thereof.

10. Oil based dispersion according to any one of claims 6 to 9, wherein the oil based dispersion comprises

> i) at least one thickener selected from the group comprising silicate such as magnesium silicate, aluminium silicate, silica dimethylsilicate, hydrophobic fumed silica, polyacrylic acid, salts of polyacrylic acid, derivatives of polyacrylic acid, PEG compounds such as PEG-8 myristate, PEG-30 glyceryl cocoate, PEG-80 glyceryl cocoate, PEG-15 soyamide/IPDI copolymer, PEG-40 sorbitan peroleate, PEG-150 stearate and mixtures thereof, methyl cellulose, ethyl cellulose, propyl cellulose, carboxymethylcellulose, xanthan gum, ammonium acryloyldimethyltaurate/VP copolymer and mixtures thereof, and/or
> ii) at least one emollient selected from the group comprising isocetylstearoylstearate, ethylhexyl stearate, octyldodecyl stearoyl stearate, isocetyl stearate, isopropyl isostearate, isostearyl isostearate, ethylhexyl hydroxystearate, ethylhexyl palmitate, isopropyl palmitate, neopentyl glycol diheptanoate, ethylhexyl isononanoate, isononyl isononanoate, cetearyl isononanoate, cetearyl octanoate, diisopropyl adipate, dicapryl adipate, diisostearylmalate, decyl oleate, isodecyl oleate, diisopropyl myristate, isostearyl neopentanoate, octyl dodecyl neopentanoate, ethylhexyl cocoate, PEG-7 glyceril cocoate, C12-15 alkyl benzoate, C16-17 alkyl benzoate, stearyl benzoate, isostearyl benzoate, ethylhexyl benzoate, octyldodecyl benzoate, cocoglyceride, coconut alkanes, coco-caprylate/caprate and mixtures thereof, and/or
> iii) at least one oil selected from the group comprising alkanecoconutester, polydimethylsiloxanes, polyalkylmethylsiloxanes, silicones, vegetable oils such as palm oil, esters of vegetable oils and mixtures thereof.

11. Base formulation consisting of

> i) a water based dispersion, as defined in any one of claims 1 to 5, in an amount from 0.0 to 100.0 wt.-%, based on the total weight of the base formulation, and/or
> ii) an oil based dispersion, as defined in any one of claims 6 to 10, in an amount from 0.0 to 100.0 wt.-%, based on the total weight of the base formulation.

12. Base formulation according to claim 11, wherein the base formulation is free of octocrylene as organic UV-A filter.

13. Cosmetic formulation having UV-A and/or UV-B protection, the cosmetic formulation comprising

> a) from 1.0 to 80.0 wt.-%, based on the total weight of the cosmetic formulation, of a base formulation as defined in any one of claims 11 or 12, and
> b) from 20.0 to 99.0 wt.-%, based on the total weight of the cosmetic formulation, of at least one further additive selected from the group comprising emulsifier, emollients, fragrances, colorants, skin tanning compounds, stabilizers, antioxidants, pigments, preserving agents, wetting agents, oils, water and mixtures thereof.

14. Cosmetic formulation according to claim 13, wherein the cosmetic formulation is selected from a sunscreen product, eye makeup product, facial makeup product, lip care product, hair care product, hair styling product, nail care product, hand care product, skin care product and mixtures thereof.

15. A method for preparing a cosmetic formulation having UV-A and/or UV-B protection according to any one of claims 13 or 14, the method comprises at least the steps of:

> a) providing a water based dispersion, as defined in any one of claims 1 to 5,
> b) providing an oil based dispersion, as defined in any one of claims 6 to 10,
> c) providing at least one further additive selected from the group comprising emulsifier, emollients, fragrances, colorants, stabilizers, antioxidants, pigments, preserving agents, wetting agents, oils, water and mixtures thereof, as defined in claim 13,
> d) combining the water based dispersion provided in step a) with the oil based dispersion provided in step b) such as to form a base formulation, as defined in any one of claims 11 or 12, and
> e) combining the base formulation obtained in step d) with the at least one further additive provided in step c) such that a cosmetic formulation is formed.

16. Use of a water based dispersion as defined in any one of claims 1 to 5 and/or an oil based dispersion as defined in any one of claims 6 to 10 or a base formulation as defined in any one of claims 11 or 12 in cosmetic formulations, preferably a sunscreen product, eye makeup product, facial makeup product, lip care product, hair care product, hair styling product, nail care product, hand care product, skin care product and mixtures thereof.

Europäisches
Patentamt
European
Patent Office
Office européen
des brevets

# EUROPEAN SEARCH REPORT

Application Number

EP 14 17 9746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/062310 A1 (AMCOL INTERNATIONAL CORP [US]) 15 August 2002 (2002-08-15) | 1,11-14,16 | INV. A61K8/29 |
| Y | * abstract * * Gel #2; example 9; table X * * page I * | 2-5,15 | A61Q17/04 A61K8/04 |
| X | FR 2 873 028 A1 (OREAL [FR]) 20 January 2006 (2006-01-20) | 1,11-14,16 | |
| Y | * examples 1-4 * | 2-5,15 | |
| X | FR 2 873 020 A1 (OREAL [FR]) 20 January 2006 (2006-01-20) | 1,11-14,16 | |
| Y | * examples 1-4 * | 2-5,15 | |
| Y | "Kemira UV-Titan for cosmetics", , 1 January 2006 (2006-01-01), pages 1-8, XP055063754, Retrieved from the Internet: URL:http://usuarios.advance.com.ar/4343808 8/Kemira UV-TITAN.pdf [retrieved on 2013-05-23] * Typical properties; page 3 - page 4 * | 1-5, 11-16 | |
| | | | TECHNICAL FIELDS SEARCHED (IPC) |
| | | | A61K A61Q |
| Y | DE 102 25 125 A1 (GOLDSCHMIDT AG TH [DE]; DEGUSSA [DE]) 18 December 2003 (2003-12-18) * paragraphs [0018] - [0022] * * paragraph [0046] * | 1-5, 11-16 | |
| X | WO 2007/136881 A2 (GRUNE GUERRY L [US]) 29 November 2007 (2007-11-29) * claims; examples III,IV * | 6,11-16 | |

-/--

The present search report has been drawn up for all claims

| Place of search | Date of completion of the search | Examiner |
|---|---|---|
| Munich | 29 April 2015 | Villa Riva, A |

Europäisches
Patentamt

European
Patent Office

Office européen
des brevets

# EUROPEAN SEARCH REPORT

Application Number

EP 14 17 9746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 671 677 A1 (OREAL [FR]) 21 June 2006 (2006-06-21) * paragraphs [0024], [0025] * * claims; examples 2,4,5 * ----- | 6-11, 13-16 | |
| X | EP 1 974 717 A1 (SHISEIDO CO LTD [JP]) 1 October 2008 (2008-10-01) * abstract * * Embodiments 3,4 * * claims * ----- | 6,11-16 | |
| X | FR 2 775 441 A1 (SEROBIOLOGIQUES LAB SA [FR]) 3 September 1999 (1999-09-03) * example 10 * ----- | 6-16 | |

TECHNICAL FIELDS
SEARCHED        (IPC)

The present search report has been drawn up for all claims

| Place of search | Date of completion of the search | Examiner |
|---|---|---|
| Munich | 29 April 2015 | Villa Riva, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

Europäisches
Patentamt

European
Patent Office

Office européen
des brevets

**Application Number**

EP 14 17 9746

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

**Application Number**

EP 14 17 9746

The Search Division considers that the present European patent application does not comply with the requirements of unity of invention and relates to several inventions or groups of inventions, namely:

1. claims: 1-5(completely); 11-16(partially)

   Water-based titanium dioxide dispersion; "base" and cosmetic sunscreen formulation including it and their use
   ---

2. claims: 6-10(completely); 11-16(partially)

   Oil-based titanium dioxide dispersion; "base" and cosmetic sunscreen formulation including it and their use
   ---

**ANNEX TO THE EUROPEAN SEARCH REPORT
ON EUROPEAN PATENT APPLICATION NO.**

EP 14 17 9746

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2015

| Patent document cited in search report | | Publication date | Patent family member(s) | | Publication date |
|---|---|---|---|---|---|
| WO 02062310 | A1 | 15-08-2002 | AT | 367790 T | 15-08-2007 |
| | | | AU | 2002243838 A1 | 19-08-2002 |
| | | | CA | 2437743 A1 | 15-08-2002 |
| | | | DE | 60221354 T2 | 10-04-2008 |
| | | | EP | 1357892 A1 | 05-11-2003 |
| | | | JP | 4137639 B2 | 20-08-2008 |
| | | | JP | 2004522751 A | 29-07-2004 |
| | | | KR | 20040025893 A | 26-03-2004 |
| | | | MX | 253435 B | 16-01-2008 |
| | | | PL | 364144 A1 | 13-12-2004 |
| | | | US | 2002182155 A1 | 05-12-2002 |
| | | | US | 2003152531 A1 | 14-08-2003 |
| | | | WO | 02062310 A1 | 15-08-2002 |
| FR 2873028 | A1 | 20-01-2006 | AT | 411085 T | 15-10-2008 |
| | | | EP | 1768749 A1 | 04-04-2007 |
| | | | FR | 2873028 A1 | 20-01-2006 |
| | | | JP | 2008505951 A | 28-02-2008 |
| | | | US | 2010254920 A1 | 07-10-2010 |
| | | | WO | 2006005521 A1 | 19-01-2006 |
| FR 2873020 | A1 | 20-01-2006 | FR | 2873020 A1 | 20-01-2006 |
| | | | US | 2007166248 A1 | 19-07-2007 |
| DE 10225125 | A1 | 18-12-2003 | AU | 2003232769 A1 | 22-12-2003 |
| | | | BR | 0311555 A | 12-04-2005 |
| | | | CN | 1658817 A | 24-08-2005 |
| | | | DE | 10225125 A1 | 18-12-2003 |
| | | | EP | 1511458 A2 | 09-03-2005 |
| | | | JP | 3984613 B2 | 03-10-2007 |
| | | | JP | 2005528454 A | 22-09-2005 |
| | | | KR | 20050019112 A | 28-02-2005 |
| | | | US | 2005224749 A1 | 13-10-2005 |
| | | | WO | 03103816 A2 | 18-12-2003 |
| WO 2007136881 | A2 | 29-11-2007 | EP | 2037878 A2 | 25-03-2009 |
| | | | US | 2007286826 A1 | 13-12-2007 |
| | | | WO | 2007136881 A2 | 29-11-2007 |
| EP 1671677 | A1 | 21-06-2006 | EP | 1671677 A1 | 21-06-2006 |
| | | | FR | 2879457 A1 | 23-06-2006 |
| | | | JP | 2006176516 A | 06-07-2006 |
| EP 1974717 | A1 | 01-10-2008 | CN | 101330898 A | 24-12-2008 |
| | | | EP | 1974717 A1 | 01-10-2008 |
| | | | JP | 5260060 B2 | 14-08-2013 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 14 17 9746

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2015

| Patent document cited in search report | | Publication date | Patent family member(s) | | Publication date |
|---|---|---|---|---|---|
| | | | KR 20080081925 A | | 10-09-2008 |
| | | | US 2009169496 A1 | | 02-07-2009 |
| | | | WO 2007069430 A1 | | 21-06-2007 |
| FR 2775441 | A1 | 03-09-1999 | AU | 2525699 A | 15-09-1999 |
| | | | EP | 1060011 A1 | 20-12-2000 |
| | | | FR | 2775441 A1 | 03-09-1999 |
| | | | JP | 2002504425 A | 12-02-2002 |
| | | | WO | 9943426 A1 | 02-09-1999 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2266527 A2
- US 5916544 A
- EP 1068866 A2
- US 6749838 B1
- WO 0203929 A1
- US 7470423 B2
- WO 1998052525 A1
- EP 1544256 A2

**Non-patent literature cited in the description**

- *Arch Dermatol,* 1988, vol. 124, 869-871